# EUROPÄISCHE PATENTSCHRIFT

(11) **EP 3 688 000 B1**
(45) Veröffentlichungstag und Bekanntmachung des Hinweises auf die Patenterteilung: **08.11.2023**
(21) Anmeldenummer: 18778422.8
(22) Anmeldetag: 20.09.2018
(51) Int. Cl.: C07F 3/02, C07B 49/00

(54) **KOHLENWASSERSTOFF-LÖSLICHE HALOGEN- UND THIOLAT-/MAGNESIUM-AUSTAUSCHREAGENZIEN**
HYDROCARBON-SOLUBLE HALOGEN AND THIOLATE/MAGNESIUM EXCHANGE REAGENTS
RÉACTIFS D'ÉCHANGE HALOGÈNE/MAGNÉSIUM ET THIOLATE/MAGNÉSIUM SOLUBLES DANS LES HYDROCARBURES

(30) Priorität: 27.09.2017 DE 102017217230; 18.01.2018 DE 102018200805
(43) Veröffentlichungstag der Anmeldung: 05.08.2020
(73) Patentinhaber: Albemarle Germany GmbH, 65926 Frankfurt am Main (DE); Ludwig-Maximilians-Universität München, 80539 München (DE)
(72) Erfinder: KNOCHEL, Paul, 81479 München (DE); ZIEGLER, Dorothée, 80638 München (DE); SIMON, Meike, 84489 Burghausen (DE)
(74) Vertreter: Uexküll & Stolberg
(86) Internationale Anmeldenummer: PCT/EP2018/075506
(87) Internationale Veröffentlichungsnummer: WO 2019/063418

(56) Entgegenhaltungen:
- EP-A1- 1 582 524
- SILVIA ZARAGOZA-CALERO ET AL: "Solid state and solution studies of lithium tris(n-butyl)magnesiates stabilised by Lewis donors", DALTON TRANSACTIONS, Bd. 44, Nr. 16, 1. Januar 2015 (2015-01-01), Seiten 7258-7267, XP055519288, GB ISSN: 1477-9226, DOI: 10.1039/C5DT00435G
- STEPHAN HEITZ ET AL: "Molecular Heterobimetallic Approach to Li-Containing MgO Nanoparticles with Variable Li-Concentrations Using Lithium-Methylmagnesium Alkoxide Clusters", CHEMISTRY OF MATERIALS, Bd. 22, Nr. 16, 24. August 2010 (2010-08-24), Seiten 4563-4571, XP055519314, ISSN: 0897-4756, DOI: 10.1021/cm100415m
- Dorothée S. Ziegler ET AL: "Generation of Aryl and Heteroaryl Magnesium Reagents in Toluene by Br/Mg or Cl/Mg Exchange", Angewandte Chemie International Edition, vol. 57, no. 22, 14 April 2018 (2018-04-14), pages 6701-6704, XP055519292, ISSN: 1433-7851, DOI: 10.1002/anie.201802123
- Ziegler Dorothée Sophia: "Metalation and Functionalization of Pyridones, Naphthyridones and Pyrones Using TMP-Bases and Generation of Aryl and Heteroaryl Magnesium Reagents in Toluene by Br/Mg-and Cl/Mg-Exchange", Dissertation zur Erlangung des Doktorgrades, 16 October 2018 (2018-10-16), pages 1-184, XP055780326, München Retrieved from the Internet: URL:https://edoc.ub.uni-muenchen.de/23372/ 7/Ziegler_Dorothee.pdf [retrieved on 2021-03-01]

## Beschreibung

Die Erfindung betrifft kohlenwasserstoff-lösliche Reagenzien zum Austausch von Halogen- oder Thiolatfunktionen in Aromaten oder Heteroaromaten gegen Magnesium.

Organomagnesiumhalogenide spielen eine wichtige Rolle als Schlüsselintermediate in der organischen Synthese. Seit der Entdeckung durch Victor Grignard sind diese Verbindungen von zentraler Bedeutung in der modernen organischen Synthese. Üblicherweise werden sie durch direkte Insertion von Magnesium in Form von Magnesiumspänen, aktiviertem Magnesiumpulver oder durch Magnesiumspäne in Anwesenheit von Lithiumchlorid dargestellt. Die heterogene Natur dieser Reaktionen kompliziert jedoch die Übertragung vom Labor auf die Großanlage und den industriellen Gebrauch. Funktionalisierte Grignardverbindungen können auf diese Art häufig nicht hergestellt werden, da funktionelle Gruppen wie Nitrile oder Ester angegriffen und zersetzt würden. Eine direkte Magnesierung von Arenen oder Heteroarenen kann durch den Gebrauch von in THF gut löslichen gehinderten Magnesiumamiden wie zum Beispiel TMPMgCl·LiCl oder TMP₂Mg·2LiCl (TMP = 2,2,6,6-tetramethylpiperidyl) erreicht werden. Alternativ können Organomagnesiumhalogenide durch einen Halogen-Magnesium-Austausch mittels Umsetzung von Aryl- oder Heteroaryliodiden oder -bromiden mit bestimmten Alkylmagnesiumhalogeniden (Rieke, D. R., Sell, M. S. In Handbook of Grignard reagents (Eds.: G. S. Silvermann, P. E. Rakita) 1996) oder besser mit dem *Turbo*-Grignard Reagenz (*i*-PrMgCl·LiCl) (Krasvoskiy, A.; Knochel P.; Angew. Chem. Int. Ed. 2004, 43, 3333) dargestellt werden. Die Turbo-Grignardreagenzien liegen als Lösungen in etherhaltigen Lösungsmitteln (THF usw.) vor und die weiteren Umsetzungen werden ebenfalls unter Verwendung von etherbasierten Lösungsmitteln durchgeführt.

Es sind nur wenige Methoden zur Synthese von Organomagnesiumverbindungen in apolaren Lösungsmitteln (Kohlenwasserstoffen) bekannt (Chtcheglova, L.; Carlotii, S.; Deffieux, A.; Poirier, N.; Barbier, M.; Fr Demanden 2003, FR 2840901A1; 20031219 und Baillie, E. S..; Bluemke, T. D.; Clegg, W.; Kennedy, A. R.; Klett, J.; Russo, L.; de Tullio, M.; Hevia E. Chem. Comm. 2014, 50, 12859). So werden Dialkylmagnesiumverbindungen (R₂Mg) durch direkte insertion von Magnesium in die C-Halogen-Bindungen langkettiger Alkylhalogenide, welche eine Verzweigung in alpha-Position aufweisen, dargestellt. Es ist weiterhin bekannt, dass gemischte Kalium-Magnesium at-Komplexe mit Silylsubstituenten (Me₃SiCH₂)₃MgK)darge-stellt in Benzol - als starke Basen für die Generierung von Arylmagnesiaten in Hexan ange-wendet werden können (Hevia, E. Chem. Commun. 2014, 50, 12859). Austauschreagenzien der Zusammensetzung RMgHal (R= Alkyl, Aryl; Hal = Halogen ausgewählt aus CI, Br, I) sind in donorlösemittelfreien, kohlenstoffwasserstoff-basierten Lösungsmitteln unbekannt. Es ist vielmehr bekannt, dass sich Grignardverbindungen mit ihren Disproportionierungsprodukten R₂Mg und MgCl₂ im Gleichgewicht gemäß

2 RMgHal → R₂Mg + MgHal₂

befinden ("Schlenk-Gleichgewicht"), wobei die Gleichgewichtslage von der Donizität des Lösungsmittels abhängt. In donorfreien Lösungsmitteln, also Kohlenwasserstoffen, liegen pracktisch ausschließlich die Dissoziationsprodukte Dialkylmagnesium und Magnesiumhalogenid vor, wobei das MgHal₂ infolge Unlöslichkeit ausfällt.

Halogen/Magnesium-Austauschreagenzlösungen in apolaren Lösungsmitteln wären von großem industriellen Interesse. Abgesehen davon, dass Kohlenwasserstoffe zu den preiswertesten Lösungsmitteln gehören, könnte durch den Gebrauch von Kohlenwasserstofflösungsmitteln die Produktaufarbeitung wesentlich vereinfacht und das Recycling des nicht wasserlöslichen Lösungsmittels bei wässriger Aufarbeitung verbessert werden. Kohlenwasserstoffe (KW) sind im Gegensatz zu beispielsweise Tetrahydrofuran (THF) nämlich nicht in Wasser löslich oder gar damit mischbar, so dass die zumeist organolöslichen Produkte leicht abgetrennt werden können. Weiterhin sind die wässrigen Phasen demzufolge nur mit ganz geringen Konzentrationen an organischen Verbindungen belastet, was positive Auswirkungen auf die Umweltverträglichkeit hat.

Silvia Zaragoza-Calero et al. "Solid State and solution studies of lithium tris(n-butyl)magnesiates stabilized by Lewis donors", Dalton Transactions, Bd. 44, Nr. 16, 1. Januar 2015, Seiten 7258-7267, beschreibt Lithium-tris(n-butyl)magnesium-Verbindungen, die durch Lewis-Donoren stabilisiert sind, mit der Formel Li(µ-ⁿBu)₂Mg(µ-ⁿBu)₂Mg(µ-ⁿBu)₂Li(donor), wie beispielsweise R¹MgR^{1·}LiOR^{2·}LiOR^{3·}aDonor und R¹MgOR^{3·}LiOR^{2·}aDonor.

Das Dokument EP 1 582 524 B1 beschreibt Reagenzien der allgemeinen Formel R*(MgX)ₙ · LiY , wobei:
n 1 oder 2 ist;
R* ein substituiertes oder nicht-substituiertes C₄-C₂₄-Aryl oder C₃-C₂₄-Heteroaryl, das eines oder mehrere Heteroatome wie B, O, N, S, Se, P, F, Cl, Br, I oder Si enthält; ein lineares oder verzweigtes, substituiertes oder nicht-substituiertes C₁₋C₂₀-Alkyl, C₂-C₂₀ - Alkenyl oder C₂-C₂₀ -Alkinyl; oder substituiertes oder nicht-substituiertes C₃-C₂₀-Cycloalkyl ist;
X und Y unabhängig voneinander oder jeweils CI, Br oder I, bevorzugt Cl; HalOn (wobei n = 3, 4); Carboxylat mit der Formel RCO₂; Alkoxid oder Phenoxid mit der
Formel RO; Dialkoxid mit der Formel LiO-R-O; Disilazid mit der Formel (R₃Si)₂N;
Thiolat mit der Formel SR; RP(O)O₂; oder SCOR; wobei R wie R* oben definiert ist;
lineares oder verzweigtes, substituiertes oder nicht-substituiertes C₁-C₂₀-Alkyl oder C₃-C₂₀-Cycloalkylamin mit der Formel RNH; Dialkyl/ Arylamin mit der Formel R₂N (wobei R wie oben definiert ist oder R₂N ein heterocyclisches Alkylamin darstellt);
Phosphin mit der Formel PR₂ (wobei R wie oben definiert ist oder PR₂ ein heterocyclisches Phosphin darstellt); OₙSR (wobei n = 2 oder 3 und R wie oben definiert ist); oder NOₙ (wobei n = 2 oder 3); oder X= R* wie oben definiert, sind.

Die Patentschrift beschreibt Mischungen aus Organomagnesiumverbindungen und Lithiumsalzen als Lösungen in organischen Donorlösungsmitteln. Dort werden zwar auch Kohlenwasserstoffe als Lösungsmittel beansprucht, jedoch wird nicht aufgezeigt, wie derartige KWlösliche Produkte der allgemeinen Formel R*(MgX)ₙ · LiY hergestellt und wofür sie im Speziellen verwendet werden können.

Stephan Heitz et al. "Molecular Heterobimetallic approach to Li-Containing MgO Nanoparticles with Variable Li-Concentrations Using Lithium-Methylmagnesium Alkoxide Clusters, Chemistry of Materials, Bd. 22, Nr. 16, 24. August 2010, Seiten 4563-4571, beschreibt Lithiummethylmagnesiumalkoxid-Verbindungen mit der Formel [Li(THF)(MeMg)₃(µOR)₄], in der R = iPr oder tBu ist.

Die Erfindung hat sich die Aufgabe gestellt, kohlenwasserstoff-lösliche Reagenzien zum Austausch von Halogen- oder Thiolatfunktionen gegen Magnesium, Verfahren zu deren Herstellung und deren Verwendung anzugeben, wobei diese Reagenzien insbesondere für die Metallierung von solchen Substraten, die nach bisherigem Stand der Technik mittels Halogen/Magnesium- Austauschprinzip nicht magnesiert werden können, verwendbar sein sollen. Dabei handelt es sich vor allem um elektronenreiche Aromaten und Heteroaromaten. Weiterhin sollen spezielle Ausführungsformen der kohlenwasserstoff-löslichen austauschaktiven Verbindungen auch in der Lage sein, wenig reaktive Substrate wie Chloraryle oder Thiolato-Aryle durch Austausch der Chlor- oder Thilolatfunktion zu magnesiieren.

Erfindungsgemäß wird die Aufgabe gelöst durch kohlenwasserstofflösliche Austauschreagenzien mit der allgemeinen Formel

R¹MgR¹₁₋ₙ(OR³)ₙ·LiOR²·(1-n)LiOR³ · aDonor

wobei: R¹ aus der Gruppe bestehend aus Isopropyl (i-Pr), n-Butyl (n-Bu), sec-Butyl (s-Bu), tert-Butyl (t-Bu) and n-Hexyl (n-Hex) ausgewählt ist und
OR² sowie OR³ jeweils unabhängig voneinander sein können:
   e) tert-Alkoxy-,
   f) sec-Alkoxy-,
   g) primäres Alkoxy- OCH₂CHR⁴R⁵, bestehend aus 4-12 C-Atomen, wobei der Alkoxyrest eine Verzweigung in Position 2 relativ zur O-Funktion aufweist und R⁴ sowie R⁵ unabhängig voneinander Alkyl-radikale mit 1-8 C-Atomen darstellen, oder
   h) Alkoxy-, enthaltend eine weitere Alkoxyfunktion, mit der allgemeinen Formel O(CHR⁶)_{b}OR⁷ mit R⁶ = H oder ein Alkylradikal mit 1-6 C-Atomen, welches entweder linear ist oder eine Verzweigung an der 3- oder höheren Position relativ zur O-Funktion aufweist, R⁷ ein lineares oder verzweigtes Alkylradikal mit 2-12 C-Atomen ist und b = eine ganze Zahl von 1 bis 4 ist,
wobei a einen Wert von 0 bis 2 einnimmt; n beliebige Werte von 0 bis 1 einnimmt und der Donor ein Diamin oder ein Triamin ist, wobei a bevorzugt einen Wert zwischen 0,5 und 1,5 und n bevorzugt die Werte 0 oder 1 darstellt.
Bevorzugt nimmt n die Werte 0 oder 1 ein. Diese Verbindungen sind allgemein zum Austausch von Halogen- oder Thiolatfunktionen gegen Magnesium befähigt. Es wurde überraschend gefunden, dass erfindungsgemäße Austauschreagenzien mit n = 1 eine gute bis exzellente Löslichkeit in Kohlenwasserstoffen auf-weisen, wobei diese ihre Grignardanaloge Struktur und Zusammensetzung bewahren und also nicht gemäß 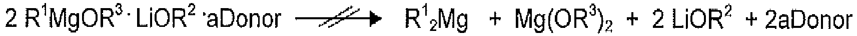 disproportionieren. Auch die Dialkylmagnesium-haltigen Reagenzien mit n = 0 weisen in der Regel eine hervorragende Löslichkeit in Kohlenwasserstoffen auf.

Als tert-Alkoxy sind tert-Butylat (OtBu), *tert*-Amylat (OtAm) und 2,3-Dimethyl-pentan-3-olat (OC(CH₃)Et(iPr)) besonders bevorzugt. Als primäres Alkoxy OCH₂CHR⁴R⁵ wird bevorzugt 2-Ethylhexanolat (OCH₂CH(Et)Bu) eingesetzt. Ein -Alkoxy kann beispielsweise OCH(CH₃)Hex sein. Als Alkoxy, enthaltend eine weitere Alkoxyfunktion sind OCH₂CH₂OBu, OCH₂CH₂OCH₂CH(Et)Bu und OCH(Me)CH₂OBu bevorzugt. Die Diaminbase ist beispielsweise Tetramethylethylendiamin (TMEDA). Als Triamin ist beispielhaft Bis(2-dimethylaminoethyl)methylamin (PMDETA) zu nennen.

Besonders bevorzugt ist, dass die Austauschreagenzien als Lösungen mit einer Konzentration von mindestens 0,5 mol/kg bezogen auf Mg in einem kohlenwasserstoffhaltigen Lösungsmittel oder Lösungsmittelgemisch vorliegen, wobei die Lösungen maximal 1 Gew.-% eines etherischen Lösungsmittels enthalten.

Weiterhin bevorzugt ist, dass das Austauschreagenz als Lösung in Kohlenwasserstoffen vorliegt, wobei die Kohlenwasserstoffe ausgewählt sind aus den Gruppen bestehend aus: Aromaten und Aliphaten. Bevorzugt enthält die erfindungsgemäße Lösung lediglich Mengen von 0,001 bis 0,5 Gew.-% eines sauerstoffhaltigen Donorlösungsmittels. Besonders bevorzugte aromatische Lösungsmittel sind Toluol, Ethylbenzol und Xylole. Bevorzugte Aliphaten sind Hexan, Heptan, Octan, Cyclohexan, Methylcyclohexan sowie handelsübliche Petrolethergemische.

Die erfindungsgemäßen kohlenwasserstoff-löslichen Reagenzien zum Austausch von Halogen- oder Thiolatfunktionen gegen Magnesium der allgemeinen Formel R¹MgR¹₁₋ₙ(OR³)ₙ LiOR² · (1-n) LiOR³ · aDonor werden verwendet für Austauschreaktionen mit halogenierten Aromaten oder Heteroaromaten der allgemeinen Formeln Hal-Ar und Hal-HetAr sowie Thiolaten der allgemeinen Formeln R⁸S-Ar und R⁸S-HetAr gemäß nachfolgenden Reaktionsgleichungen:
für n = 1:
R¹MgOR³ · LiOR²· aDonor + Hal-Ar → R¹-Hal + Ar-Mg-OR³ . LiOR²· aDonor oder
R¹MgOR³ · LiOR²· aDonor + Hal-HetAr → R¹-Hal + HetAr-Mg-OR³ · LiOR^{2.} aDonor für n = 0:
R¹MgR¹· LiOR^{2.} LiOR³· aDonor + 2 Hal-Ar → 2 R¹-Hal + Ar₂Mg . LiOR²· LiOR³ · aDonor oder
R¹MgR¹· LiOR² · LiOR³· aDonor + 2 Hal-HetAr → 2 R¹-Hal + HetAr₂Mg . LiOR^{2.} LiOR³ · aDonor
Dieselben Gleichungen gelten analog für Thiolat-funktionalisierte Substrate (ersetze in die= sem Fall Hal durch SR⁸).

Die halogenierten bzw. thiolatfunktionalisierten Aromaten oder Heteroaromaten können eine oder mehrere funktionelle Gruppen besitzen, _auswählt aus der Gruppe besteht aus: F, CI, Br, CN, CO₂R, OR, OH, NR₂, NHR, NH₂, PR₂, P(O)R₂, CONR₂, CONHR, SR, SH, CF₃, NO₂. Im allgemeinen werden Austauschreagenzien mit n = 1 zum Austausch von Br, I oder SR⁸ - Gruppen eingesetzt, während die reaktiveren Dialkylmagnesium-basierten Reagenzien mit n = 0 auch zur Magnesiierung von Chlorfunktionen in Aromaten oder Heteroaromaten verwendet werden können. Besonders leicht der Magnesiierung zugängliche Chloraromaten und Chlorheteroaromaten besitzen mindestens eine Alkoxyfunktion in benachbarter Stellung zum Chlor. Beispiele sind: 2-Chloranisol; 1,2-Chlormethoxynaphthalin; 2,3-Chlormethoxynaphtha-lin; 1,5-Dichlor-2-methoxy-4,6-dimethylbenzol, wobei unter Magneiisierung der Austausch der Aromaten oder Heteroaromaten verstanden wird. Besonders bevorzugt werden die kohlenwasserstoff-löslichen Halogen/Magnesium-Austauschreagenzien für Austauschreaktionen mit elektronenreichen halogenierten Aromaten oder Heteroaromaten der allgemeinen Formeln Hal-Ar bzw Hal-HetAr verwendet. Elektronenreiche Aromaten und Heteroaromaten werden nämlich mit den bisher bekannten Austauschreagenzien in THF-Lösung nur unvollständig oder nur sehr langsam angegriffen (L. Shi, Y. Chu, P. Knochel, H. Mayr, J. Org. Chem. 2009, 74, 2760; L. Shi, Y. Chu, P. Knochel, H. Mayr, Org. Lett. 2009, 11, 3502):

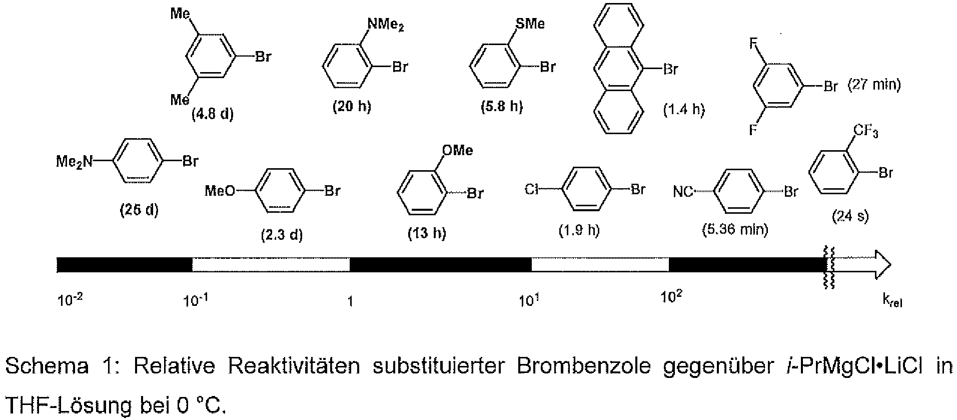

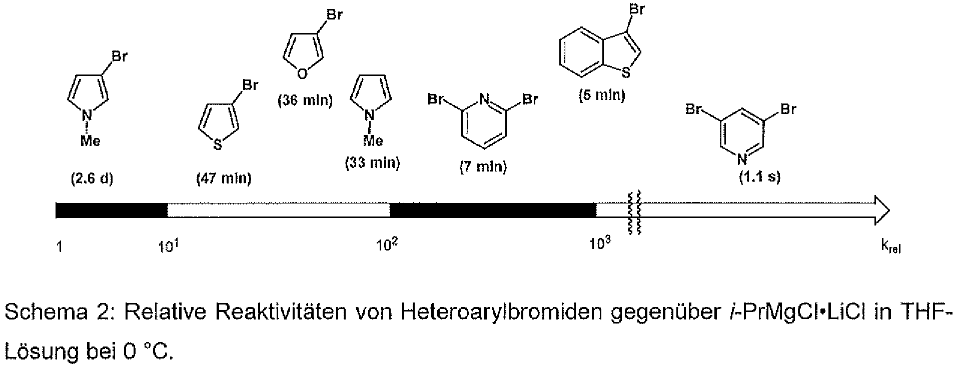

Reaktionszeiten > 1h sind für praktische Anwendungen nachteilig oder prohibitiv. Zu den elektronenreichen Aromaten gehören solche Verbindungen, die induktiv elektronenschiebende Gruppen (z.B. Alkylgruppen, Phenylgruppen) oder mesomer schiebende Gruppen (z.B. -NR₂, -NHR, NH₂, -OH, -OR, -NHC(O)-R u.ä.) aufweisen. Zu den elektronenreichen Heteroaromaten gehören beispielsweise Fünfringverbindungen wie Pyrrole, Furane, Thiophene, Oxazole, Isoxazole, Thiazole, Isothiazole, Imidazole, Benzimidazole, Triazole, Indazole, Indole u.ä.

Besonders bevorzugte Beispiele sind die halogenierten Aromaten oder Heteroaromaten auswählt aus der Gruppe bestehend aus: Brombenzol, Bromtoluolen, Bromanisolen, Brom-N,N-dimethylanilinen, 1-Brom-3,5-dimethoxybenzol, Bromnaphthalinen, Bromphenanthrenen, Bromthiophenen, Brompyridinen, Brombenzothiophenen, Brombenzofuranen, 1,2-Dibromcyclopent-1-en, 2-Chloranisol, 1,2-Chlormethoxynaphthalin, 2,3-Chlormethoxynaphthalin, 1,5-Dichlor-2-methoxy-4,6-dimethylbenzol.

Die erfindungsgemäßen Austauschreagenzien werden bevorzugt auch zum Thiolat/Magnesium-Austausch von schwefelfunktionalisierten, stickstoffhaltigen Heteroatomaten eingesetzt. Beispiele sind: *Tert*-butyl 2-(methylthio)piperidin-1-carboxylat, *Tert*-butyl 2-(phenylthio)piperidin-1-carboxylat, *Tert*-butyl 4-methyl-2-(phenylthio)piperidin-1-carboxylat, *Tert*-butyl 2-((4-methoxyphenyl)thio)piperidin-1-carboxylat, *Tert*-butyl 2-((4-fluorophenyl)thio)piperidin-1-carboxylat, *Tert*-butyl 2-(phenylthio)pyrrolidin-1-carboxylat, 2-(Phenylthio)pyridin.

Es wurde überraschend gefunden, dass die erfindungsgemäßen in Toluol gelösten Austauschreagenzien beispielsweise das als Testsystem verwendete 4-Bromanisol innerhalb 15 Minuten (min) bei Raumtemperatur praktisch vollständig zu metallieren befähigt sind, während mit den aus dem Dokument EP1582524 B1 bekannten Austauschreagenzien in THF kein nennenswerter Umsatz beobachtet wird. Es werden die GC-Ausbeuten nach Umsetzung mit dem Elektrophil H₂O bestimmt:

**Tabelle 1: Brom/Magnesium-Austauschreaktionen an 4-Bromanisol mit Austauschreagenzien nach bekanntem Stand der Technik (Nr. 1-12) und erfindungsgemäßem s- BuMgOCH₂CH(Et)Bu · LiOCH₂CH(Et)Bu in Toluol (Nr. 13-14)**

| 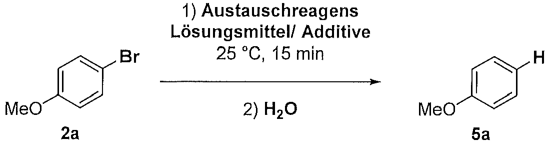 | | | | |
|---|---|---|---|---|
| Nr. | Austauschreagens | Lösungsmittel | TMEDA (Äquiv.) | GC Ausbeute (%) |
| 1 | *i*-PrMgCl·LiCl in THF (1.2 Äquiv.) | THF | 0 | 0.8 |
| 2 | *i*-PrMgCl·LiCl in THF (1.2 Äquiv.) | THF- | 1.2 | 1.7 |
| 3 | *i*-PrMgCl·LiCl in Toluol (1.2 Äquiv.) | Toluol | 0 | 0 |
| 4 | *i*-PrMgCl·LiCl in Toluol (1.2 Äquiv.) | Toluol | 1.2 | 0 |
| 5 | *s*-Bu₂Mg·LiCl in THF (0.6 Äquiv.) | THF | 0 | 13 |
| 6 | *s*-Bu₂Mg·LiCl in THF (0.6 Äquiv.) | THF | 1.2 | 13 |
| 7 | *s*-Bu₂Mg·LiCl in Toluol (0.6 Äquiv.) | Toluol | 0 | 0 |
| 8 | *s*-Bu₂Mg·LiCl in Toluol (0.6 Äquiv.) | Toluol | 1.2 | 0 |
| 9 | *s*-BuMgCl·LiCl in THF (1.2 Äquiv.) | THF | 0 | 2 |
| 10 | *s*-BuMgCl·LiCl in THF (1.2 Äquiv.) | THF | 1.2 | 3 |
| 11 | s-BuMgCl·LiCl in Toluol (1.2 Äquiv.) | Toluol | 0 | 0 |
| 12 | *s*-BuMgCl·LiCl in in Toluol (1.2 Äquiv.) | Toluol | 1.2 | 0 |
| 13 | *s*-BuMgOCH₂CH(Et)Bu ·LiOCH₂CH(Et)Bu in Toluol (1.2 Äquiv.) | Toluol | 0 | 85 |
| 14 | *s*-BuMgOCH₂CH(Et)Bu ·LiOCH₂CH(Et)Bu in Toluol (1.2 Äquiv.) | Toluol | 1.2 | 99 |

Es wurden verschiedene kohlenwasserstofflösliche Austauschreagenzien untersucht. Produkte mit Pprimären und sekundären Alkoholatrestene zeigten gegenüber 4-Bromanisol etwa ähnliche Umsätze, während solche mit tertiären Alkoholaten etwas langsamer in ihrem Reaktionsverhalten sind. Eine Ausnahme bildet hierbei das 5-Nonanol (-OCHBu₂), welches aufgrund von sterischer Hinderung bei den milden Reaktionsbedingungen offensichtlich zu keiner Reaktion führte (Tabelle 2).

**Tabelle 2: Austauschreaktionen an 4-Bromanisol mit verschiedenen Austauschreagenzien (Variation des Alkoholrests)**

| 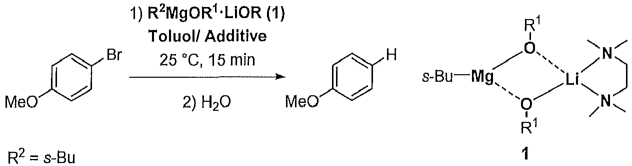 | | | |
|---|---|---|---|
| Nr. | R1 | | GC Ausbeute (%) |
| 1 | CH(Bu)₂ | | 0 |
| 2 | C(CH₃)(Et)(*i*-Pr) | | 83 |
| 3 | CH(Me)Hex | | 99 |
| 4 | CH₂CH(Et)Bu | | 99 |
| 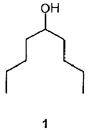 | 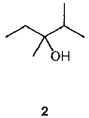 | 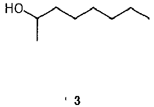 | 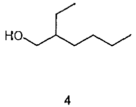 |

Als besonders vorteilhaftes Austauschreagenz erwies sich sec-BuMgOCH₂CH(Et)Bu·LiOCH₂CH(Et)Bu, welches für die Metallierung verschiedener funktionalisierter Brombenzole getestet wurde (Tabelle 3).

**Tabelle 3: Halogen-Magnesium Austausch an Arylverbindungen**

| 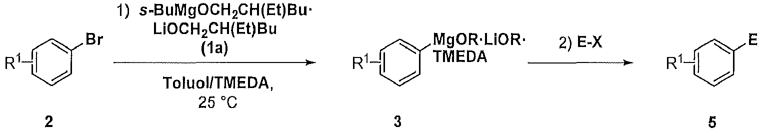 | | | | |
|---|---|---|---|---|
| Nr. | Edukt | E (Äquiv.) | Zeit (Min.) | Produkt/Ausbeute^{a} |
| 1 | 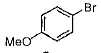 2a | H₂O a (1.2) | 15 | 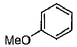 5a, 96% |
| 2 | 2a | I₂ b (1.2) | 15 | 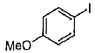 5b, 70% |
| 3 | 2a | MeSSMe c (1.2) | 15 | 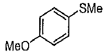 5c, 93% |
| 4 | 2a | 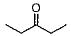 d (1.2) | 15 | 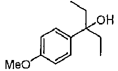 Sd, 80% |
| 5 | 2a | 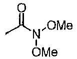 e (1.2) | 15 | 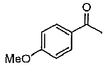 Se, 92% |
| 6 | 2a | 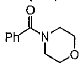 f (1.2) | 15 | 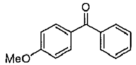 5f, 70% |
| 7 | 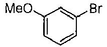 2b | I₂ a (1.2) | 15 | 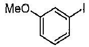 5g, 65% |
| 8 | 2b | MeSSMe c (1.2) | 15 | 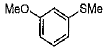 5h, 96% |
| 9 | 2b | 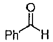 g (1.2) | 15 | 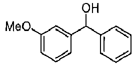 5i, 99% |
| 10 | 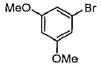 2c | 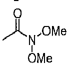 e (1.2) | 15 | 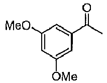 5j, 89% |
| 11 | 2c | 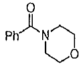 f (1.2) | 15 | 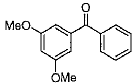 5k, 67% |
| 12 | 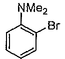 2d | MeSSMe c (1.2) | 60 | 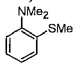 51,84% |
| 13 | 2d | 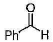 g (1.2) | 60 | 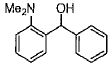 Sm, 80% |
| 14 | 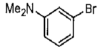 2e | MeSSMe c (1.2) | 90 | 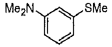 Sn, 76% |
| 15 | 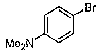 | 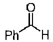 | 240 | 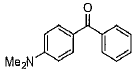 |

Dasselbe Reagenz wurde auch für den Mg/Br-Austausch an Heteroarylverbindungen getestet (Tab. 4).

**Tabelle 4: Halogen-Magnesium Austausch an Heteroarylverbindungen**

| 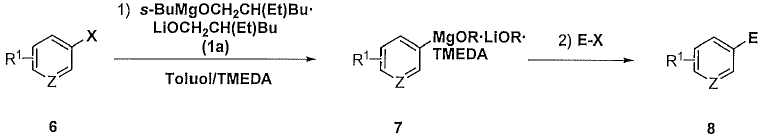 Z=O,S | | | | |
|---|---|---|---|---|
| X= Br, Cl | | | | |
| Nr. | Edukt | E (Äquiv.) | Zeit (Min.) | Produkt/ Ausbeute^{a} |
| 1 | 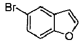 6a | 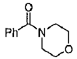 (1.2) | 10 | 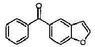 8a; 70%^{b} |
| 2 | 6a | (1.2) | 10 | 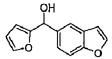 **8b;** 74%^{b} |
| 3 | 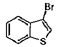 6b | 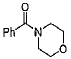 (1.2) | 10 | 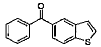 8c; 65%^{c} |
| 4 | 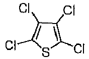 6c | 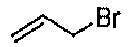 a (2.6) | 240 | 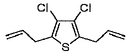 8d, 60%^{b} |

| | | | | |
|---|---|---|---|---|
| Ausbeute des analytisch reinen Produkts. ^{b}Die Reaktionen wurden bei 25 °C durchgeführt. ^{c}Die Reaktion wurde bei -10 °C durchgeführt. | | | | |

Das folgende Schema 3 zeigt die Ergebnisse der Verwendung der metallierten Aromaten für Kreuzkupplungsreaktionen:.

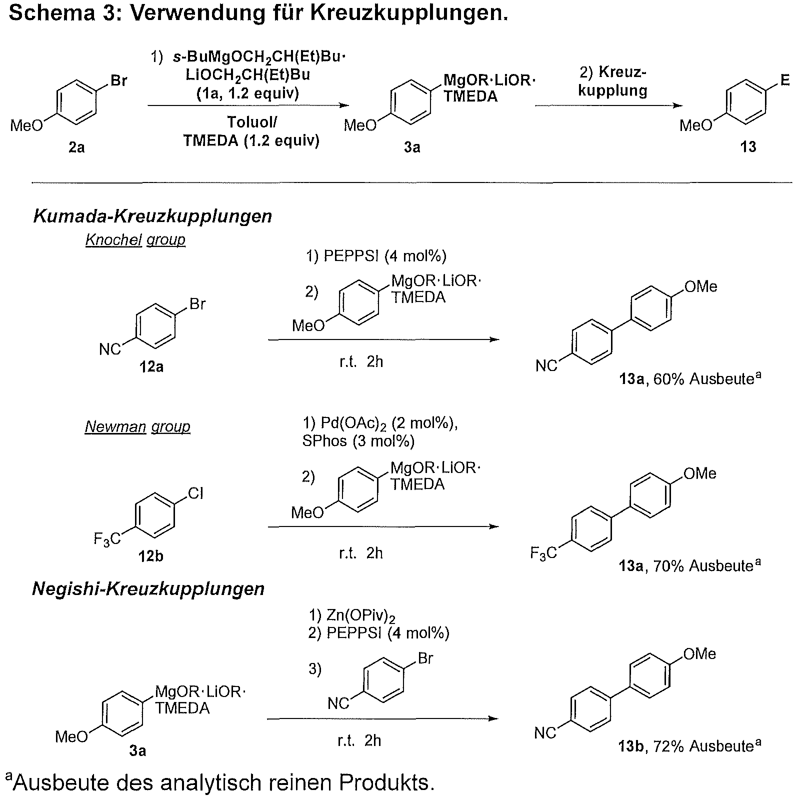

Weiterhin können die kohlenwasserstoff-löslichen Halogen/Magnesium-Austauschreagenzien zur Deprotonierung terminaler Alkine verwendet werden. Durch Umsetzung mit Elektrophilen, beispielsweise Carbonylverbindungen, lassen sich organische Zwischenprodukte, bevorzugt Carbinole, erhalten. Für die Deprotonierung wurde Ethinyltoluol als Testsystem eingesetzt (Schema 4):

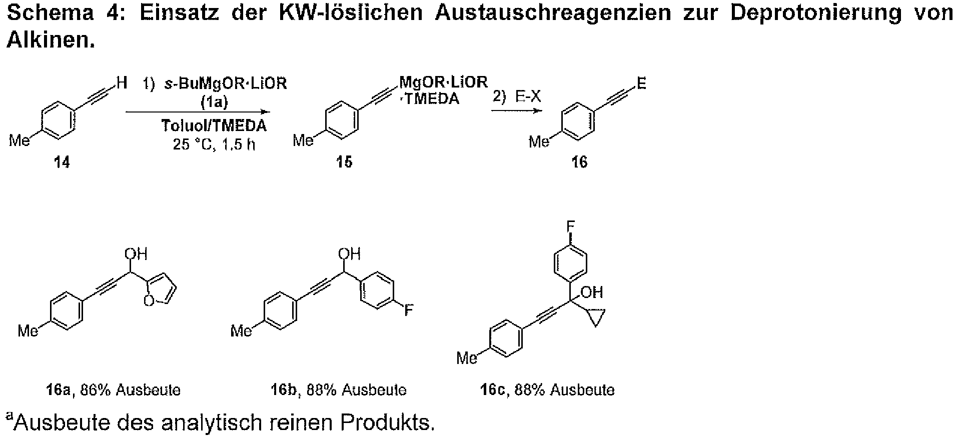

Die erfindungsgemäßen Austauschreagenzien werden durch die nachfolgenden drei alternativen Verfahren erhalten, indem
1) eine Dialkoxymagnesiumverbindung R²O-Mg-OR³ mit (n+1) Äquivalenten einer Alkyllithiumverbindung R¹Li in einem kohlenwasserstoffhaltigen Lösungsmittel oder Lösungsmittelgemisch umgesetzt wird, wobei
   R¹ aus der Gruppe bestehend aus Isopropyl (i-Pr), n-Butyl (n-Bu), sec-Butyl (s-Bu), tert-Butyl (t-Bu) and n-Hexyl (n-Hex) ausgewählt ist,
   n bevorzugt die Werte 0 oder 1 einnimmt und
   OR² und OR³ unabhängig voneinander sein können:
      a) tert-Alkoxy,
      b) sec-Alkoxy,
      c) primäres Alkoxy OCH₂CHR⁴R⁵, bestehend aus 4-12 C-Atomen, wobei der Alkoxyrest eine Verzweigung in Position 2 relativ zur O-Funktion aufweist und R⁴ sowie R⁵-unabhängig voneinander Alkylradikale mit 1-8 C-Atomen darstellen, oder
      d) Alkoxy, enthaltend eine weitere Alkoxyfunktion, mit der allgemeinen Formel O(CHR⁶)OR⁷ mit R⁶ = H oder ein Alkylradikal mit 1-6 C-Atomen, welches entweder linear ist oder eine Verzweigung an der 3- oder höheren Position relativ zur O-Funktion aufweist, R⁷ ein lineares oder verzweigtes Alkylradikal mit 2-12 C-Atomen ist und b = ein ganze Zahl von 1 bis 4 ist

         R²O-Mg-OR³ + (n+1) R¹-Li → R¹MgR¹₁₋ₙ(OR³)ₙ· LiOR² · (1-n) LiOR³
      oder
2) eine Dialkylmagnesiumverbindung R¹-Mg-R⁹ in einem kohlenwasserstoffhaltigen Lösungsmittel oder Lösungsmittelgemisch für n = 1 mit einem Äquivalent eines Alkohols R³OH sowie einem Äquivalent einer Lithiumalkoxidverbindung R²OLi umgesetzt wird bzw. für n = 0 insgesamt zwei Äquivalente an Lithiumalkoxidverbindungen R²OLi und/oder R³OLi zugegeben werden mit R⁹ = Alkyl bestehend aus 1-8 C-Atomen, gleich oder verschieden zu R¹, ansonsten gilt die gleiche Definition der Substituenten R¹ bis R⁸ wie vorstehend beschrieben:
   1a.

      R¹-Mg-R⁹ + R³-OH → R¹-Mg-OR³ + HR⁹
   1b.

      R¹-Mg-OR³ + LiOR² --> R¹-Mg-OR³ · LiOR²
   2.

      R¹-Mg-R⁹ + LiOR² + LiOR³ -7 R¹-Mg-R⁹ . LiOR² · LiOR³

      oder
   3) eine Dialkylmagnesiumverbindung R¹-Mg-R⁹ mit einem Äquivalent einer Dialkoxymagnesiumverbindung R⁵O-Mg-OR³ in einem kohlenwasserstoffhaltigen Lösungsmittel oder Lösungsmittelgemisch umgesetzt wird und zu diesem Reaktionsgemisch 0,5 - 1,5 Äquivalente einer Lithiumalkoxidverbindung R²OLi zugegeben werden, auch mit der Definition der Substituenten R¹ bis R⁸ wie vorstehend beschrieben.

   1.

      R¹MgR⁹ + R⁵O-Mg-OR³ → 2 [(R³O)_{0,5}(R⁵O)_{0,5})]Mg[R¹₀,R⁹₀,₅]
   2.

      [(R³O)_{0,5}(R⁵O)_{0,5})]Mg[R¹_{0,}R⁹_{0,5}] + LiOR² → [(R³O)_{0,5}(R⁵O)_{0,5})]Mg[R¹₀,R⁹_{0,5}] · LiOR²

Es gelten somit folgende Definitionen der Substituenten:
- R und R*: ein substituiertes oder nicht-substituiertes C₄-C₂₄-Aryl oder C₃-C₂₄-Heteroaryl, das eines oder mehrere Heteroatome wie B, O, N, S, Se, P, F, Cl, Br, I oder Si enthält; ein lineares oder verzweigtes, substituiertes oder nicht-substituiertes C₁-C₂₀-Alkyl, C₂-C₂₀-Alkenyl oder C₂-C₂₀-Alkinyl; oder substituiertes oder nicht-substituiertes C₃-C₂₀-Cycloalkyl
- R¹: ausgewählt aus der Gruppe bestehend aus Isopropyl (i-Pr), n-Butyl (n-Bu), sec-Butyl (s-Bu), tert-Butyl (t-Bu) and n-Hexyl (n-Hex)
- R⁴, R⁵, R⁹: eine Alkylgruppe bestehend aus 1-8 C-Atomen

- R⁶: H oder ein Alkylradikal mit 1-6 C-Atomen, welches entweder linear ist oder eine Verzweigung an der 3- oder höheren Position relativ zur O-Funktion aufweist
- R⁷: lineares oder verzweigtes Alkylradikal mit 2-12 C-Atomen
- R⁸: Alkyl oder Aryl, bevorzugt Phenyl ist
- OR² sowie OR³: gleich oder verschieden sind und primäre, sekundäre oder tertiäre Alkoxygruppen mit 3 bis 18 Kohlenstoffatomen
- Hal-Ar: halogenierte Aromaten
- Hal-HetAr: halogenierte Heteroaromaten
- R⁸S-Ar: Arylthiolat
- R⁸S-HetAr: Hetroarylthiolat

Das Verfahren kann noch dadurch verbessert werden, dass dem Reaktionsgemisch aus der Synthese a Äquivalente eines Donors, bezogen auf Mg, zugegeben wird, wobei der Donor ein Diamin oder ein Triamin ist und a einen Wert von 0 bis 2, bevorzugt 0,5 bis 1,5 aufweist.

Vorteilhafterweise werden bei allen Verfahren lediglich kohlenwasserstoffhaltige Lösungsmittel eingesetzt.

Die metallierten Zwischenprodukte Ar-Mg-OR³· LiOR^{2.} aDonor, HetAr-Mg-OR³· LiOR²· aDonor, Ar₂Mg · LiOR^{2.} LiOR³ · aDonor sowie HetAr₂Mg · LiOR² · LiOR³ · aDonor werden bevorzugt für CC-oder CN-Kupplungsreaktionen (*Kumada*-Typ Kreuzkupplungen, meist Pdkatalysiert) oder für Additionsreaktionen durch Umsetzung mit Elektrophilen verwendet. Als Elektrophile kommen alle mit carbanionischen Reaktionszentren reagierende Verbindungen in Frage, beispielsweise Carbonylverbindungen (Aldehyde, Ketone, Carbonsäureester, Carbonsäureamide), Nitrile, Imine, Halogene, Halogenverbindungen, Disulfide, Wasser u.a.

Die Erfindung wird nachfolgend an Hand von Ausführungsbeispielen näher erläutert.

### GENERELLE INFORMATIONEN

Alle Reaktionen wurden unter Argonatmosphäre in ausgeheizten Glasgeräten durchgeführt. Spritzen, welche zum Transfer von wasserfreien Lösungsmitteln oder Reagenzien verwendet wurden, wurden zuvor mit Argon gespült. Toluol und THF wurden kontinuierlich refluxiert und frisch destilliert über Natriumbenzophenon unter Stickstoffatmosphäre eingesetzt. Die Lösemittel wurden über Molsieb trocken gelagert. TMEDA und 2-Ethylhexanol wurden frisch destilliert unter Verwendung von Calciumhydrid. Die Ausbeuten beziehen sich auf die isolierten Verbindungen, welche zu geschätzt >95% rein sind, ermittelt durch ¹H-NMR (25 °C) und Gaschromatographie (GC). Mittels Säulenchromatographie wurden die Verbindungen aufgereinigt, wobei Silicagel von Merck verwendet wurde (SiO₂ 0.040-0.063 mm, 230-400 mesh ASTM).

NMR-Spektren wurden in CDCl₃ gemessen und chemische Verschiebungen wurden in parts per million (ppm) gemessen. Abkürzungen für Signalkopplungen sind wie folgt: s, singlet; d, doublet; t, triplet; q, quartet; m, multiplet; br, broad. Massenspektren und High Resolution Mass Spectra (HRMS) wurden mittels Elektronenionisation (EI) ermittelt. GC-Spektren wurden mit Hilfe von Maschinen des Typs Hewlett-Packard 6890 oder 5890 Series II (Hewlett Packard, 5 % Phenylmethylpolysiloxan; Länge: 10 m, Durchmesser: 0.25 mm; Filmdicke: 0.25 µm) gemessen. Alle Reagenzien wurden von kommerziell erhältlichen Quellen bezogen. Magnesium-2-ethylhexanolat wurde von Albermarle (Frankfurt/Hoechst) erhalten.

### Beispiel 1: Herstellung von sec-BuMgOCH₂CH(Et)Bu·OLiCH₂CH(Et)Bu aus Magnesium bis(2-ethylhexanolat) und sec-BuLi

Ein trockener und mit Argon gefüllter Schlenk-Kolben, welcher mit einem Magnetrührstäbchen und einem Septum ausgestattet wurde, wurde mit Mg[OCH₂CH(Et)Bu]₂ (0.85 M in Heptan, 15.0 mL, 12.8 mmol) befüllt und die Reaktionsmischung wurde auf 0 °C gekühlt. Anschließend wurde s-BuLi (1.21 M in Hexan, 10.6 mL, 12.8 mmol) tropfenweise hinzugefügt. Nach komplettem Hinzufügen wurde die Reaktionsmischung auf 25°C gebracht und die Reaktionslösung für 2 Stunden gerührt. Hierbei entstand eine leicht gelbliche Lösung. Anschließend wurde das Lösungsmittel unter Vakuum abgedampft, wobei ein leicht gelber Schaum entstand. Frisch destilliertes Toluol (ca. 9 mL) wurde unter ständigem Rühren bei 0 °C hinzugefügt. Das auf diese Art und Weise hergestellte
*s*-BuMgOCH₂CH(Et)Bu·OLiCH₂CH(Et)Bu wurde anschließend bei 0 °C iodometrisch titriert.

Die Konzentration der resultierenden klaren Lösung entsprach einer Molarität von 0.80-1.3 M.

### Beispiel 2: Herstellung von sec-BuMgOCH₂CH(Et)Bu·OLiCH₂CH(Et)Bu aus Dibutylmagnesium, 2-Ethylhexanol und sec-BuLi

Ein trockener und mit Argon gefüllter *Schlenk-*Kolben, welcher mit einem Magnetrührstäbchen und einem Septum ausgestattet wurde, wurde mit *n*-Bu₂Mg (0.66 M in Hexan, 15.0 mL, 9.9 mmol) befüllt. Anschließend wurde unter Eiskühlung bei 0°C 2-Ethylhexanol (3.1 mL, 19.8 mmol) langsam hinzugetropft. Hierbei entstand unter Wärmeentwicklung eine gelartige Verbindung. Anschließend wurde diese bei 0°C mit s-BuLi (1.21 M in Hexan, 8.18 mL, 9.9 mmol) versetzt. Nach komplettem Hinzufügen wurde die Reaktionsmischung auf 25°C gebracht und die Reaktionslösung für 2 Stunden gerührt. Hierbei löste sich die gelartige Verbindung und es entstand eine leicht gelbliche Lösung. Anschließend wurde das Lösungsmittel unter Vakuum abgedampft, wobei ein leicht gelber Schaum entstand. Frisch destilliertes Toluol (ca. 1 mL) wurde unter ständigem Rühren bei 0 °C hinzugefügt.

### Beispiel 3: Herstellung von n-BuMgOCH₂CH(Et)Bu·OLiCH₂CH(Et)Bu

Ein trockener und mit Argon gefüllter *Schlenk-*Kolben, mit einem Magnetrührstäbchen und einem Septum ausgestattet, wurde mit Mg[OCH₂CH(Et)Bu]₂ (0.85 M in Heptan, 15.0 mL, 12.8 mmol) befüllt und die Reaktionsmischung wurde auf 0 °C gekühlt. Anschließend wurde *n*-BuLi (2.1 M in Hexan, 6.1 mL, 12.8 mmol) tropfenweise hinzugefügt. Nach komplettem Hinzufügen wurde die Reaktionsmischung auf 15°C gebracht und die Reaktionslösung für 2 Stunden gerührt. Hierbei entstand eine leicht gelbliche Lösung. Anschließend wird das Lösungsmittel unter Vakuum abgedampft, wobei ein leicht gelber Schaum entsteht. Frisch destilliertes Toluol (ca. 9 mL) wurde unter ständigem Rühren bei 0 °C hinzugefügt.

### Beispiel 4: Herstellung von sec-Bu₂Mg . 2 LiOCH₂CH(Et)Bu . PMDTA aus Dibutylmagnesium, 2-Ethylhexanol und sec-BuLi in Toluol (??)

Ein trockener und mit Argon gefüllter Schlenk-Kolben, welcher mit einem Magnetrührstäbchen und einem Septum ausgestattet wurde, wurde mit *n*-Bu₂Mg (0.66 M in Hexan, 15.0 mL, 9.9 mmol) befüllt. Anschließend wurde unter Eiskühlung bei 0°C 2-Ethylhexanol (3.1 mL, 19.8 mmol) langsam hinzugetropft. Hierbei entstand unter Wärmeentwicklung eine gelartige Verbindung. Anschließend wurde diese bei 0°C mit s-BuLi (1.21 M in Hexan, 16.36 mL, 19.8 mmol) versetzt. Nach komplettem Hinzufügen wurde die Reaktionsmischung auf 25°C gebracht und die Reaktionslösung für 2 Stunden gerührt. Hierbei löste sich die gelartige Verbindung und es entstand eine leicht gelbliche Lösung. Anschließend wurde das Lösungsmittel unter Vakuum abgedampft, wobei ein leicht gelber Schaum entstand. Frisch destilliertes Toluol (ca. 1 mL) wurde unter ständigem Rühren bei 0 °C hinzugefügt.

### Beispiel 5: Herstellung von sec-Bu₂Mg · 2 LiOCH₂CH(Et)Bu aus Magnesium bis(2-ethylhexanolat) und sec-BuLi

Ein trockener und mit Argon gefüllter Schwenk-Kolben, welcher mit einem Magnetrührstäbchen und einem Septum ausgestattet wurde, wurde mit Mg[OCH²CH(Et)Bu]₂ (0.85 M in Heptan, 15.0 mL, 12.8 mmol) befüllt und die Reaktionsmischung wurde auf 0°C gekühlt. Anschließend wurde sec-BuLi (1.21 M in Hexan, 21.2 mL, 25.6 mmol) tropfenweise hinzugefügt. Nach komplettem Hinzufügen wurde die Reaktionsmischung auf 25°C gebracht und die Reaktionslosung für 2 Stunden gerührt. Hierbei entstand eine leicht gelbliche Lösung. Anschließend wurde das Lösungsmittel unter Vakuum abgedampft, wobei ein leicht gelber Schaum entstand. Frisch destilliertes Toluol (ca. 9 mL) wurde unter ständigem Rühren bei 0 °C hinzugefügt. Das auf diese Art und Weise hergestellte
*s*-BuMg₂ ·2LiOCH₂CH(Et)Bu wurde anschließend bei 0 °C iodometrisch titriert. Die Konzentration der resultierenden klaren Lösung entsprach einer Molarität von 0.60-0.85 M.

### Beispiel 6: Typische Verfahrensweise für die Herstellung von Aryl- und Heteroarylmagnesiumalkoxidverbindungen durch einen Brom-Magnesium Austausch

Ein trockener und mit Argon gefüllter Kolben, ausgestattet mit einem Magnetrührstäbchen und einem Septum, wird mit dem jeweiligen Aryl- bzw. Heteroarylbromid (1.0 eq.) befüllt und in trockenem Toluol (0.5 M Lösung) und TMEDA (1.2 eq.) gelöst. Die resultierende Lösung wird bei der jeweils angegebenen Temperatur gerührt und
*sec*-BuMgOCH₂CH(Et)Bu·OLiCH₂CH(Et)Bu (1.2 eq.) wird hinzugetropft. Das Ende des Brom-Magnesium Austausches wird mittels GC-Analyse von mit Wasser gequenchten Aliqouts überprüft, wobei als interner Standard Tetradecan verwendet wird. Nachfolgend werden die Aryl- bzw. Heteroarylmagnesiumalkoxidverbindungen mit Elektrophilen unter angegebenen Bedingungen umgesetzt. Nach vollständigen Umsetzungen werden die Reaktionsmischungen mit gesättigter wässriger NH₄Cl Lösung gequencht und mit EtOAc (3 x 20 mL) extrahiert. Die vereinten organischen Phasen werden über Na₂SO₄ getrocknet, filtriert und konzentriert.

### Beispiel 7: Typische Verfahrensweise für die Herstellung von Diaryl- und Di-heteroarylmagnesiumverbindungen durch einen Chor-Magnesium Austausch

Ein trockener und mit Argon gefüllter Kolben, ausgestattet mit einem Magnetrührstäbchen und einem Septum, wird mit dem jeweiligen Aryl- bzw. Heteroarylhalogen (1.0 eq.) befüllt und in trockenem Toluol gelöst (ca. 0.5 M Losung) und mit PMDTA (0.6 eq.) versetzt. Die resultierende Lösung wird bei der jeweils angegebenen Temperatur gerührt und *sec*-Bu₂Mg · 2 LiOCH₂CH(Et)Bu (0.6 eq.) wird hinzugetropft. Das Ende des Brom-Magnesium Austausches wird mittels GC-Analyse von mit Wasser gequenchten Aliquots überprüft, wobei als interner Standard Tetradecan verwendet wird. Im Anschluft werden die Aryl- bzw. Heteroarylmagnesiumalkoxidverbindungen mit Elektrophilen unter angegebenen Bedingungen umgesetzt. Nach vollständigem Umsatz werden die Reaktionsmischungen mit gesättigter wässriger NH₄Cl Lösung gequencht und mit EtOAc (3 x 20 mL) extrahiert. Die vereinten organischen Phasen werden über Na₂SO₄ getrocknet, filtriert und konzentriert.

### Beispiel 8: Herstellung von (2,5-Dimethoxyphenyl)(phenyl)methanol

Gemäß der in Beispiel 7 angegebenen Vorgehensweise wurde zu einer Mischung aus 2-Chlor-1,4-dimethoxybenzol (0.14 mL, 1.00 mmol), PMDTA (0.12 mL, 0.6 mmol) und Toluol (1 mL) *sec*-BuMg₂ ·2 LiOCH₂CH(Et)Bu (0.76 M in Toluol, 0.79 mL, 0.6 mmol, 0.6 eq.) bei 25 °C hinzugetropft. Nach 45 Minuten wurde Benzaldehyd (0.1 mL, 1.0 mmol, 1.2 eq.) hinzugefügt und die Reaktionsmischung wurde für weitere 60 Minuten bei 25°C gerührt. Die Aufreinigung des Rohprodukts erfolgte mittels Säulenchromatographie (Silicagel, /-Hexan/Ethylacetat 9:1) und lieferte die Titelverbindung als farbloses Öl (149 mg, 0.61 mmol, 61%).

¹H-NMR (400 MHz, CDCl₃): δ / ppm = 7.47 - 7.40 (m, 2H), 7.35 (t, J = 7.4, 2H), 7.30 - 7.25 (m, 1H), 6.89 (d, J = 2.9, 1H), 6.87 - 6.78 (m, 2H), 6.04 (d, J = 4.1, 1H), 3.77 (d, J = 3.6, 6H), 3.12 (d, J = 4.8, 1H).

¹³C-NMR(101 MHz, CDCl₃): δ / ppm = 153.8, 151.0, 143.2, 133.2, 128.2, 127.3, 126.6, 114.1, 112.8, 111.9, 72.3, 56.0, 55.7.

MS (EI, 70 eV): *m*/*z* (%) = 244 (100), 226 (11), 167 (13), 165 (15), 139 (45), 105 (30), 91 (14), 79 (12), 77 (28), 43 (37).

HRMS (EI): *m*/*z* calc. for [C₁₅H₁₆O₃]: 244.1099; found: 244.1095. 35

IR(Diamond-ATR, neat): *ṽ* / cm"1 =2938, 2834, 1591, 1492, 1452, 1276, 1212, 1177, 1037, 831.

### Beispiel 9: Typische Verfahrensweise für die Herstellung von Heteroarylmagnesiumalkoxid-Verbindungen durch einen Thiolat-Magnesium Austausch:

Ein trockener und mit Argon gefüllter Glaskolben, ausgestattet mit einem Magnetrührstäbchen und einem Septum, wird mit dem jeweiligen thiolatfunktionalisierten Heteroaren (1.0 eq.) befüllt, in trockenem Toluol gelöst (0.5 M Lösung) und mit ca. 3 eq TMEDA versetzt. Die resultierende Lösung wird bei Raumtemperatur gerührt und *sec*-BuMgOCH₂CH(Et)Bu · LiOCH₂CH(Et)Bu (3.0 eq.) wird hinzugetropft (0.05 mL/min). Das Ende des Thiolat-Magnesium Austausches wird mittels GC-Analyse von mit Wasser gequenchten Aliqots überprüft, wobei als interner Standard Undecan verwendet wird. Nachfolgend werden die Heteroarylmagnesiumalkoxidverbindungen mit Elektrophilen unter angegebenen Bedingungen umgesetzt. Nach vollständiger Umsetzung werden die Reaktionsmischungen mit gesättigter wässriger NH₄Cl-Lösung gequencht und mit Et₂O (3 x 20 mL) extrahiert. Die vereinten organischen Phasen werden über Na₂SO₄ getrocknet, filtriert und konzentriert.

### Beispiel 10: Herstellung von Tert-butyl 2-allyl-4-methylpiperidin-1-carboxylat

Gemäß der in Beispiel 9 angegebenen Vorgehensweise wurde zu einer Mischung aus *Tert-butyl* 2-(methylthio)piperidin-1-carboxylat (92 mg, 0.30 mmol), TMEDA (0.134 mL, 0.9 mmol) und Toluol (0.6 mL) *sec*-BuMgOCH₂CH(Et)Bu · LiOCH₂CH(Et)Bu (1.10 M in Toluol, 0.82 mL, 0.9 mmol 3.0 eq.) bei 25°C hinzugetropft (0.05 mL/min). Nach 4 Stunden wurde Allylbromid (78 µL, 0.9 mmol, 3.0 eq.) bei 0 °C hinzugefügt, das Reaktionsgemisch wurde auf -40 °C gekühlt und mit CuCN · 2 LiCI-Lösung (1.0 M in THF, 0.09 mL, 0.09 mmol, 0.3 eq.) versetzt und anschließend wurde für weitere 14 Stunden bei 25°C gerührt. Die Aufreinigung des Rohprodukts erfolgte mittels Säulenchromatographie (Silicagel, *i*-Hexan/Diethylether 95:5) und lieferte die Titelverbindung als farbloses Öl (59 mg, 0.25 mmol, 83%).

¹H-NMR (599 MHz, CDCl₃): δ / ppm = 5.77 (ddt, J = 17.2, 10.2, 7.2 Hz, 1H), 5.08-4.97 (m, 2H), 3.85 (tt, J = 8.3, 6.3 Hz, 1H), 3.73 (ddd, J = 13.9, 7.3, 3.2 Hz, 1H), 3.01 (ddd, J = 13.9, 10.3, 5.9 Hz, 1H), 2.40 (dddt, J = 14.2, 7.1, 5.9, 1.4 Hz, 1H), 2.24 (dtt, J = 13.5, 7.6, 1.1 Hz, 1H), 1.87 (ddtd, J = 13.3, 10.3, 7.2, 1.2 Hz, 1H), 1.73- 1.68 (m, 1H), 1.68- 1.62 (m, 1H), 1.45 (s, 8H), 1.20-1.12 (m, 1H), 1.12-1.06 (m, 1H), 0.98 (d, J = 6.8 Hz, 3H).

¹³C-NMR(101 MHz, CDCl₃): δ / ppm = 155.4, 135.4, 116.7, 79.0, 53.0, 39.0, 37.4, 34.9, 31.1, 28.4, 26.1, 21.5.

MS (EI, 70 eV): *m*/*z* (%) = 166 (3), 143 (8), 142 (100), 98 (46), 57 (3), 56 (5)..

HRMS (EI): *m*/*z* calc. for [C₁₀H₁₆ON]: 166.1232; found: 166.1225.

IR (Diamond-ATR, neat): *ṽ* / cm-1 = 2976, 2928, 2872, 1688, 1642, 1478, 1456, 1408, 1392, 1364, 1350, 1332, 1304, 1278, 1246, 1178, 1148, 1094, 1070, 992, 912, 866, 770.

### Beispiel 11: Typische Verfahrensweise für die Herstellung von Alkinylmagnesiumalkoxid-Verbindungen durch Deprotonierung:

Ein trockener und mit Argon gefüllter Kolben, ausgestattet mit einem Magnetrührstäbchen und einem Septum, wird mit dem jeweiligen Alkin (1.0 eq.) befüllt und in trockenem Toluol (0.5 M Lösung) und TMEDA (1.2 eq.) gelöst. Die resultierende Lösung wird bei der jeweils angegebenen Temperatur gerührt und *s*-BuMgOCH₂CH(Et)Bu·OLiCH₂CH(Et)Bu (1.2 eq.) wird hinzugetropft. Das Ende der Deprotonierung wird mittels GC-Analyse von mit Wasser gequenchten Aliqots überprüft, wobei als interner Standard Tetradecan verwendet wird. Nachfolgend werden die Alkinylmagnesiumalkoxidverbindungen mit Elektrophilen unter angegebenen Bedingungen (siehe Schema 4) umgesetzt. Nach vollständiger Umsetzung werden die Reaktionsmischungen mit gesättigter wässriger NH₄Cl-Lösung gequencht und mit EtOAc (3 x 20 mL) extrahiert. Die vereinten organischen Phasen werden über Na₂SO₄ getrocknet, filtriert und konzentriert.

### Beispiel 12: Herstellung von 4-lodoanisol

Gemäß der in Beispiel 7 der allgemeinen Vorschrift, Beispiel 4 angegebenen Vorgehensweise wurde zu einer Mischung aus 4-Bromoanisol (0.06 mL, 0.50 mmol), TMEDA (0.09 mL, 0.6 mmol) und Toluol (1 mL) *sec*-BuMgOCH₂CH(Et)Bu·OLiCH₂CH(Et)Bu (0.80 M in Toluol, 0.75 mL, 0.6 mmol 1.2 eq.) bei 25°C hinzugetropft. Nach 15 Minuten wurde lod (152 mg in 1 mL THF, 0.6 mmol, 1.2 eq.) hinzugefügt und die Reaktionsmischung wurde für weitere 30 Minuten bei 25 °C gerührt. Die Aufreinigung des Rohprodukts erfolgte mittels Säulenchromatographie (Silicagel, *i*-Hexan/Ethylacetat 9:1) und lieferte die Titelverbindung als weißen Feststoff (81 mg, 0.35 mmol, 70%).

¹H-NMR (400 MHz, CDCl₃): δ / ppm = 7.63 - 7.49 (d, 2H), 6.76 - 6.61 (d, 2H), 3.78 (s, 3H). ¹³C-NMR (101 MHz, CDCl₃): δ / ppm = 159.6, 138.3, 116.5, 82.8, 55.5.

MS (EI, 70 eV): *m*/*z* (%) = 234 (100), 191 (15), 92 (64).

HRMS (EI): *m*/*z* calc. for [C₇H₇IO]: 233.9542; found: 233.9540.

IR (Diamond-ATR, neat): *ṽ* / cm-1 = 3006, 2966, 2938, 2837, 1586, 1569, 1486, 1456, 1444, 1436, 1397, 1287, 1248, 1179, 1175, 1102, 1028, 999, 833, 829, 813. 8

## Patentansprüche

1. Kohlenwasserstofflösliche Austauschreagenzien mit der allgemeinen Formel
R¹MgR¹₁₋ₙ(OR³)ₙ·LiOR²· (1-n)LiOR³ · aDonor
wobei:
R¹ aus der Gruppe bestehend aus Isopropyl (i-Pr), n-Butyl (n-Bu), sec-Butyl (s-Bu), tert-Butyl (t-Bu) and n-Hexyl (n-Hex) ausgewählt ist und
OR² sowie OR³ jeweils unabhängig voneinander sein können:
e) tert-Alkoxy-,
f) sec-Alkoxy-,
g) primäres Alkoxy- OCH₂CHR⁴R⁵, bestehend aus 4-12 C-Atomen, wobei der Alkoxyrest eine Verzweigung in Position 2 relativ zur O-Funktion aufweist und R⁴ sowie R⁵ unabhängig voneinander Alkylradikale mit 1-8 C-Atomen darstellen, oder
h) Alkoxy-, enthaltend eine weitere Alkoxyfunktion, mit der allgemeinen Formel O(CHR⁶)_{b}OR⁷ mit R⁶ = H oder ein Alkylradikal mit 1-6 C-Atomen, welches entweder linear ist oder eine Verzweigung an der 3- oder höheren Position relativ zur O-Funktion aufweist, R⁷ ein lineares oder verzweigtes Alkylradikal mit 2-12 C-Atomen ist und b = eine ganze Zahl von 1 bis 4 ist,
wobei a einen Wert von 0 bis 2 einnimmt; n beliebige Werte von 0 bis 1 einnimmt und der Donor ein Diamin oder ein Triamin ist, wobei a bevorzugt einen Wert zwischen 0,5 und 1,5 und n bevorzugt die Werte 0 oder 1 darstellt.

2. Austauschreagenzien nach Anspruch 1, **dadurch gekennzeichnet, dass** a einen Wert zwischen 0,5 und 1,5 und n die Werte 0 oder 1 darstellt.

3. Austauschreagenzien nach Anspruch 1 oder 2, **dadurch gekennzeichnet, dass** diese in Lösung mit einer Konzentration von mindestens 0,5 mol/kg bezogen auf Mg in einem kohlenwasserstoffhaltigen Lösungsmittel oder Lösungsmittelgemisch vorliegen, wobei die Lösung maximal 1 Gew.-% eines etherischen Lösungsmittels enthält.

4. Austauschreagenzien nach einem der Ansprüche 1 bis 3, **dadurch gekennzeichnet, dass** die Kohlenwasserstoffe ausgewählt sind aus den Gruppen bestehend aus:
Aromaten und Aliphaten.

5. Verfahren zur Herstellung der kohlenwasserstofflöslichen Austauschreagenzien gemäß einem der Ansprüche 1 bis 4, **dadurch gekennzeichnet, dass** eine Dialkoxymagnesiumverbindung R²O-Mg-OR³ mit (n+1) Äquivalenten einer Alkyllithiumverbindung R¹Li in einem kohlenwasserstoffhaltigen Lösungsmittel oder Lösungsmittelgemisch umgesetzt wird, wobei
R¹ aus der Gruppe bestehend aus Isopropyl (i-Pr), n-Butyl (n-Bu), sec-Butyl (s-Bu), tert-Butyl (t-Bu) and n-Hexyl (n-Hex) ausgewählt ist,
n bevorzugt die Werte 1 oder 0 einnimmt und
OR² und OR³ unabhängig voneinander sein können:
e) tert-Alkoxy-,
f) sec-Alkoxy-,
g) primärer Alkoxy- OCH₂CHR⁴R⁵, bestehend aus 4-12 C-Atomen, wobei der Alkoxyrest eine Verzweigung in Position 2 relativ zur O-Funktion aufweist und R⁴ sowie R⁵ unabhängig voneinander Alkylradikale mit 1-8 C-Atomen darstellen, oder
h) Alkoxyrest, enthaltend eine weitere Alkoxyfunktion, mit der allgemeinen Formel O(CHR⁶)_{b}OR⁷ mit R⁶ = H oder ein Alkylradikal mit 1-6 C-Atomen, welches entweder linear ist oder eine Verzweigung an der 3- oder höheren Position relativ zur O-Funktion aufweist, R⁷ ein lineares oder verzweigtes Alkylradikal mit 2-12 C-Atomen ist und b = eine ganze Zahl von 1 bis 4 ist.

6. Verfahren zur Herstellung der kohlenwasserstofflöslichen Austauschreagenzien gemäß einem der Ansprüche 1 bis 4, **dadurch gekennzeichnet, dass** eine Dialkylmagnesiumverbindung R¹-Mg-R⁹ für n = 1 mit einem Äquivalent Alkohol R³OH sowie mit einem Äquivalent einer Lithiumalkoxidverbindung R²OLi bzw. für n=0 mit insgesamt zwei Äquivalenten der Lithiumalkoxidverbindungen R²OLi und/oder R³OLi in einem kohlenwasserstoffhaltigen Lösungsmittel oder Lösungsmittelgemisch umgesetzt wird, wobei
R¹ aus der Gruppe bestehend aus Isopropyl (i-Pr), n-Butyl (n-Bu), sec-Butyl (s-Bu), tert-Butyl (t-Bu) and n-Hexyl (n-Hex) ausgewählt ist und
OR² und OR³ unabhängig voneinander sein können:
a) tert-Alkoxy-,
b) sec-Alkoxy-,
c) primärer Alkoxy- OCH₂CHR⁴R⁵, bestehend aus 4-12 C-Atomen, wobei der Alkoxyrest eine Verzweigung in Position 2 relativ zur O-Funktion aufweist und R⁴ sowie R⁵ unabhängig voneinander Alkylradikale mit 1-8 C-Atomen darstellen, oder
d) Alkoxyrest, enthaltend eine weitere Alkoxyfunktion, mit der allgemeinen Formel O(CHR⁶)_{b}OR⁷ mit R⁶ = H oder ein Alkylradikal mit 1-6 C-Atomen, welches entweder linear ist oder eine Verzweigung an der 3- oder höheren Position relativ zur O-Funktion aufweist, R⁷ ein lineares oder verzweigtes Alkylradikal mit 2-12 C-Atomen ist und b = eine ganze Zahl von 1 bis 4 ist und
R⁹ eine beliebige Alkylgruppe mit 1-8 C-Atomen ist und R⁹ entweder gleich wie oder verschieden von R¹ ist.

7. Verfahren zur Herstellung der kohlenwasserstofflöslichen Austauschreagenzien der allgemeinen Formel R¹MgOR³·LiOR²· aDonor gemäß einem der Ansprüche 1 bis 4, **dadurch gekennzeichnet, dass** eine Dialkylmagnesiumverbindung R¹-Mg-R⁹ mit einem Äquivalent einer Dialkoxymagnesiumverbindung R⁵O-Mg-OR³ in einem kohlenwasserstoffhaltigen Lösungsmittel oder Lösungsmittelgemisch umgesetzt wird, um eine Reaktionsmischung zu bilden, und zu diesem Reaktionsgemisch 0,5 - 1,5 Äquivalente einer Lithiumalkoxidverbindung R²OLi zugegeben werden, wobei
R¹ aus der Gruppe bestehend aus Isopropyl (i-Pr), n-Butyl Fassung)(n-Bu), sec-Butyl (s-Bu), tert-Butyl (t-Bu) and n-Hexyl (n-Hex) ausgewählt ist und
OR² und OR³ unabhängig voneinander sein können:
a) tert-Alkoxy-,
b) sec-Alkoxy-,
c) primärer Alkoxy- OCH₂CHR⁴R⁵, bestehend aus 4-12 C-Atomen, wobei der Alkoxyrest eine Verzweigung in Position 2 relativ zur O-Funktion aufweist und R⁴ sowie R⁵ unabhängig voneinander Alkylradikale mit 1-8 C-Atomen darstellen, oder
d) Alkoxyrest, enthaltend eine weitere Alkoxyfunktion, mit der allgemeinen Formel O(CHR⁶)_{b}OR⁷ mit R⁶ = H oder ein Alkylradikal mit 1-6 C-Atomen, welches entweder linear ist oder eine Verzweigung an der 3- oder höheren Position relativ zur O-Funktion aufweist, R⁷ ein lineares oder verzweigtes Alkylradikal mit 2-12 C-Atomen ist und b = eine ganze Zahl von 1 bis 4 ist.

8. Verfahren nach einem der Ansprüche 5 bis 7, **dadurch gekennzeichnet, dass** dem Reaktionsgemisch aus der Synthese a Äquivalente eines Donors, bezogen auf Mg, zugegeben wird, wobei a bevorzugt einen Wert von 0,5 bis 1,5 aufweist.

9. Verfahren nach einem der Ansprüche 5 bis 8, **dadurch gekennzeichnet, dass** lediglich kohlenwasserstoffbasierte Lösungsmittel eingesetzt werden.

10. Verwendung der kohlenwasserstofflöslichen Halogen- bzw. Thiolat-/Magnesium-Austauschreagenzien der allgemeinen Formel R¹MgR¹₁₋ₙ(OR³)ₙ·LiOR²·(1-n)LiOR³·aDonor gemäß Anspruch 1 bis 4 oder erhalten gemäß Anspruch 5 bis 9 für Austauschreaktionen mit halogenierten oder thiolatfunktionalisierten Aromaten oder Heteroaromaten der allgemeinen Formeln Hal-Ar, Hal-HetAr, R⁹S-Ar oder R⁹S-HetAr mit R⁹ = Alkyl oder Aryl und Einsatz der metallierten Zwischenprodukte Ar-Mg-OR³·LiOR²·aDonor, HetAr-Mg-OR³·LiOR²·aDonor, Ar₂Mg·LiOR²·LiOR³·aDonor sowie HetAr₂Mg·LiOR²·LiOR³·aDonor für CC- oder CN-Kupplungsreaktionen oder Additionsreaktionen durch Umsetzung mit Elektrophilen.

11. Verwendung der kohlenwasserstofflöslichen Halogen- bzw. Thiolat-/Magnesium-Austauschreagenzien der allgemeinen Formel R¹MgR¹₁₋ₙ(OR³)ₙ·LiOR²·(1-n)LiOR³·aDonor nach Anspruch 10 für Austauschreaktionen mit halogenierten Aromaten oder Heteroaromaten der allgemeinen Formeln Hal-Ar bzw. Hal-HetAr oder thiolatfunktionalisierten Aromaten oder Heteroaromaten der allgemeinen Formeln R⁸S-Ar bzw. R⁸S-HetAr, wobei die halogenierten oder thiolatfunktionalisierten Aromaten oder Heteroaromaten eine oder mehrere zusätzliche funktionelle Gruppen besitzen, auswählt aus der Gruppe bestehend aus: F, Cl, Br, CN, CO₂R, OR, OH, NR₂, NHR, NH₂, PR₂, P(O)R₂, CONR₂, CONHR, SR, SH, CF₃, NO₂.

12. Verwendung der kohlenwasserstofflöslichen Halogen- bzw. Thiolat-/Magnesium-Austauschreagenzien der allgemeinen Formel R¹MgR¹₁₋ₙ(OR³)ₙ·LiOR²·(1-n)LiOR³·aDonor nach Anspruch 11 für Austauschreaktionen mit elektronenreichen Aromaten.

13. Verwendung der kohlenwasserstofflöslichen Halogen- bzw. Thiolat-/Magnesium-Austauschreagenzien der allgemeinen Formel R¹MgR¹₁₋ₙ(OR³)ₙ·LiOR²·(1-n)LiOR³·aDonor nach Anspruch 11 für Austauschreaktionen mit elektronenreichen Heteroaromaten, ausgewählt aus der Gruppe bestehend aus Pyrrolen, Furanen, Thiophenen, Oxazolen, Isoxazolen, Thiazolen, Isothiazolen, Imidazolen, Benzimidazolen, Triazolen, Indazolen, Indolen.

14. Verwendung der kohlenwasserstofflöslichen Halogen- bzw. Thiolat-/Magnesium-Austauschreagenzien der allgemeinen Formel R¹MgR¹₁₋ₙ(OR³)ₙ·LiOR²·(1-n)LiOR³·aDonor nach Anspruch 12 für Austauschreaktionen mit halogenierten Aromaten oder Heteroaromaten der allgemeinen Formeln Hal-Ar bzw Hal-HetAr, wobei die halogenierten Aromaten oder Heteroaromaten auswählt sind aus der Gruppe bestehend aus: Brombenzol, Bromtoluenen, Bromanisolen, Brom-N,N-dimethyl-anilinen, 1-Brom-3,5-dimethoxybenzol, Bromnaphthalenen, Bromphenanthrenen, Bromthiophenen, Brompyridinen, Brombenzothiophenen, Brombenzofuranen, 1,2-Dibromcyclopent-1-en, *Tert*-butyl 2-(methylthio)piperidin-1-carboxylat, *Tert-*butyl 2-(phenylthio)piperidin-1-carboxylat, *Tert*-butyl 4-methyl-2-(phenylthio)piperidin-1-carboxylat, *Tert*-butyl 2-((4-methoxyphenyl)thio)piperidin-1-carboxylat, *Tert*-butyl 2-((4-fluorophenyl)thio)piperidin-1-carboxylat, *Tert-*butyl 2-(phenylthio)pyrrolidin-1-carboxylat, 2-(Phenylthio)pyridin.

## Claims

1. Hydrocarbon-soluble exchange reagents of the general formula
R¹MgR¹₁₋ₙ (OR³) ₙ · LiOR² · (1-n) LiOR³ · aDonor
in which:
R¹ is selected from the group consisting of isopropyl (i-Pr), n-butyl (n-Bu), sec-butyl (s-Bu), tert-butyl (t-Bu) and n-hexyl (n-Hex) and
OR² as well as OR³ may independently represent:
e) tert-alkoxy,
f) sec-alkoxy,
g) primary alkoxy OCH₂CHR⁴R⁵, consisting of 4-12 C-atoms, wherein the alkoxy residue has a branch at position 2 relative to the O-function and R⁴ as well as R⁵ independently represent alkyl radicals having 1-8 C-atoms, or
h) alkoxy, containing another alkoxy function, of the general formula O(CHR⁶)_{b}OR⁷ wherein R⁶ = H or an alkyl radical having 1-6 C-atoms, which is either linear or has a branch at position 3 or higher relative to the O-function, R⁷ is a linear or branched alkyl radical having 2-12 C-atoms, and b is an integer from 1 to 4, wherein a assumes a value of 0 to 2, n assumes any value from 0 to 1, and the donor is a diamine or a triamine, wherein a preferably represents a value between 0.5 and 1.5 and n preferably represents the values 0 or 1.

2. The exchange reagents according to claim 1, **characterized in that** a represents a value between 0.5 and 1.5 and n represents the values 0 or 1.

3. The exchange reagents according to claim 1 or 2, **characterized in that** these are present in solution in a hydrocarbon-containing solvent or solvent mixture in a concentration of at least 0.5 mol/kg in relation to Mg, wherein the solution contains at maximum 1 wt% of an ethereal solvent.

4. The exchange reagents according to any one of claims 1 to 3, **characterized in that** the hydrocarbons are selected from the groups consisting of: aromatics and aliphates.

5. A method for preparing the hydrocarbon-soluble exchange reagents according to any one of claims 1 to 4, **characterized in that** a dialkoxy magnesium compound R²O-Mg-OR³ is reacted with (n+1) equivalents of an alkyl lithium compound R¹Li in a hydrocarbon-containing solvent or solvent mixture, wherein
R¹ is selected from the group consisting of isopropyl (i-Pr), n-butyl (n-Bu), sec-butyl (s-Bu), tert-butyl (t-Bu) and n-hexyl (n-Hex),
n preferably assumes the values 1 or 0, and
OR² and OR³ may independently represent:
a) tert-alkoxy,
b) sec-alkoxy,
c) primary alkoxy OCH₂CHR⁴R⁵, consisting of 4-12 C-atoms, wherein the alkoxy residue has a branch at position 2 relative to the O-function and R⁴ as well as R⁵ independently represent alkyl radicals having 1-8 C-atoms, or
d) alkoxy residue, containing another alkoxy function, of the general formula O(CHR⁶)_{b}OR⁷ wherein R⁶ = H or an alkyl radical having 1-6 C-atoms, which is either linear or has a branch at position 3 or higher relative to the O-function, R⁷ is a linear or branched alkyl radical having 2-12 C-atoms, and b is an integer from 1 to 4.

6. A method for preparing hydrocarbon-soluble exchange reagents according to any one of claims 1 to 4, **characterized in that** a dialkyl magnesium compound R¹-Mg-R⁹ is reacted, for n = 1, with one equivalent alcohol R³OH as well as one equivalent of a lithium alkoxide compound R²OLi, or, for n = 0, with a total of two equivalents of the lithium alkoxide compounds R²OLi and/or R³OLi in a hydrocarbon-containing solvent or solvent mixture, wherein R¹ is selected from the group consisting of isopropyl (i-Pr), n-butyl (n-Bu), sec-butyl (s-Bu), tert-butyl (t-Bu) and n-hexyl (n-Hex) and
OR² and OR³ may independently represent:
a) tert-alkoxy,
b) sec-alkoxy,
c) primary alkoxy OCH₂CHR⁴R⁵, consisting of 4-12 C-atoms, wherein the alkoxy residue has a branch at position 2 relative to the O-function and R⁴ as well as R⁵ independently represent alkyl radicals having 1-8 C-atoms, or
d) alkoxy residue, containing another alkoxy function, of the general formula O(CHR⁶)_{b}OR⁷ wherein R⁶ = H or an alkyl radical having 1-6 C-atoms, which is either linear or has a branch at position 3 or higher relative to the O-function, R⁷ is a linear or branched alkyl radical having 2-12 C-atoms, and b is an integer from 1 to 4, and
R⁹ is any alkyl group having 1-8 C-atoms and R⁹ is either the same as, or different from R¹.

7. A method for preparing hydrocarbon-soluble exchange reagents of the general formula R²MgOR³ · LiOR² · aDonor according to any one of claims 1 to 4, **characterized in that** a dialkyl magnesium compound R¹-Mg-R⁹ is reacted with one equivalent of a dialkoxy magnesium compound R⁵O-Mg-OR³ in a hydrocarbon-containing solvent or solvent mixture to form a reaction mixture, and 0.5 to 1.5 equivalents of a lithium alkoxide compound R²OLi are added to this reaction mixture, wherein
R¹ is selected from the group consisting of isopropyl (i-Pr), n-butyl (n-Bu), sec-butyl (s-Bu), tert-butyl (t-Bu) and n-hexyl (n-Hex), and
OR² and OR³ may independently represent:
a) tert-alkoxy,
b) sec-alkoxy,
c) primary alkoxy OCH₂CHR⁴R⁵, consisting of 4-12 C-atoms, wherein the alkoxy residue has a branch at position 2 relative to the O-function and R⁴ as well as R⁵ independently represent alkyl radicals having 1-8 C-atoms, or
d) alkoxy residue, containing another alkoxy function, of the general formula O(CHR⁶)_{b}OR⁷ wherein R⁶ = H or an alkyl radical having 1-6 C-atoms, which is either linear or has a branch at position 3 or higher relative to the O-function, R⁷ is a linear or branched alkyl radical having 2-12 C-atoms, and b is an integer from 1 to 4.

8. The method according to any one of claims 5 to 7, **characterized in that** to the reaction mixture from synthesis a equivalents of donor are added in relation to Mg, and a preferably has a value of 0.5 to 1.5.

9. The method according to any one of claims 5 to 8, **characterized in that** only hydrocarbon-based solvents are used.

10. Use of the hydrocarbon-soluble halogen or thiolate/magnesium exchange reagents of the general formula R¹MgR¹₁₋ₙ (OR³) ₙ · LiOR² · (1-n) LiOR³ · aDonor according to claims 1 to 4 or obtained according to claims 5 to 9 for exchange reactions with halogenated or thiolate-functionalized aromatics or heteroaromatics of the general formulas Hal-Ar, Hal-HetAr, R⁹S-Ar, or R⁹S-HetAr wherein R⁹ = alkyl or aryl and use of the metalated intermediates Ar-Mg-OR³ · LiOR² · aDonor, HetAr-Mg-OR³ · LiOR² · aDonor, Ar₂Mg LiOR² · LiOR³ · aDonor as well as HetAr₂Mg · LiOR² · LiOR³ aDonor for CC or CN coupling reactions or addition reactions by reacting them with electrophiles.

11. The use of the hydrocarbon-soluble halogen or thiolate/magnesium exchange reagents of the general formula R¹MgR¹₁₋ₙ (OR³) ₙ · LiOR² · (1-n) LiOR³ · aDonor according to claim 10 for exchange reactions with halogenated or thiolate-functionalized aromatics or heteroaromatics of the general formulas Hal-Ar or Hal-HetAr, respectively, or thiolate-functionalized aromatics or heteroaromatics of the general formulas R⁸S-Ar or R⁸S-HetAr, respectively, wherein the halogenated or thiolate-functionalized aromatics or heteroaromatics have one or several functional groups selected from the group consisting of: F, Cl, Br, CN, CO₂R, OR, OH, NR₂, NHR, NH₂, PR₂, P(O)R₂, CONR₂, CONHR, SR, SH, CF₃, NO₂.

12. The use of the hydrocarbon-soluble halogen or thiolate/magnesium exchange reagents of the general formula R¹MgR¹₁₋ₙ (OR³) ₙ · LiOR² · (1-n) LiOR³ · aDonor according to claim 11 for exchange reactions with electron-rich aromatics.

13. The se of the hydrocarbon-soluble halogen or thiolate/magnesium exchange reagents of the general formula R¹MgR¹₁₋ₙ (OR³) ₙ · LiOR² · (1-n) LiOR³ · aDonor according to claim 11 for exchange reactions with electron-rich heteroaromatics selected from the group consisting of pyrroles, furans, thiophenes, oxazoles, isoxazoles, thiazoles, isothiazoles, imidazoles, benzimidazoles, triazoles, indazoles, indoles.

14. Use of the hydrocarbon-soluble halogen or thiolate/magnesium exchange reagents of the general formula R¹MgR¹₁₋ₙ(OR³)ₙ · LiOR² · (1-n) LiOR³ · aDonor according to claim 12 for exchange reactions with halogenated aromatics or heteroaromatics of the general formulas Hal-Ar or Hal-HetAr, respectively, wherein the halogenated aromatics or heteroaromatics are selected from the group consisting of: *bromobenzene,* bromotoluenes, bromoanisoles, bromo-N,N-dimethylanilines, 1-bromo-3,5-dimethoxybenzene, bromonaphthalenes, bromophenanthrenes, bromothiophenes, bromopyridines, bromobenzothiophenes, bromobenzofurans, 1,2-dibromocyclopent-1-ene, tert-butyl 2-(methylthio)piperidine-1-carboxylate, *tert*-butyl 2-(phenylthio)piperidine-1-carboxylate, *tert*-butyl 4-methyl-2-(phenylthio)piperidine-1-carboxylate, *tert*-butyl 2-((4-methoxyphenyl)thio)-piperidine-1-carboxylate, *tert*-butyl 2-((4-fluorophenyl)-thio)piperidine-1-carboxylate, *tert-*butyl 2-(phenylthio)-pyrrolidine-1-carboxylate, 2-(phenylthio)pyridine.

## Revendications

1. Réactifs d'échange solubles dans des hydrocarbures, de formule générale
R¹MgR¹₁₋ₙ(OR³)ₙ^{·}LiOR^{2·}(1-n)LiOR^{3·}a(donneur) dans laquelle
- R¹ représente une entité choisie dans l'ensemble constitué par les groupes isopropyle (i-Pr), n-butyle (n-Bu), sec-butyle (s-Bu), tert-butyle (t-Bu) et n-hexyle (n-Hex),
- et OR² et OR³ peuvent chacun représenter, indépendamment l'un de l'autre,
e) un groupe alcoxy tertiaire,
f) un groupe alcoxy secondaire,
g) un groupe alcoxy primaire de formule OCH₂CHR⁴R⁵, comportant de 4 à 12 atomes de carbone, lequel groupe alcoxy présente une ramification en position 2 par rapport à l'atome fonctionnel d'oxygène, et dans laquelle formule R⁴ et R⁵ représentent, indépendamment l'un de l'autre, des groupes alkyle comportant de 1 à 8 atomes de carbone,
h) ou un groupe alcoxy portant un autre groupe fonctionnel alcoxy, de formule générale O(CHR⁶)_{b}OR⁷ dans laquelle R⁶ représente un atome d'hydrogène ou un groupe alkyle comportant de 1 à 6 atomes de carbone, qui est linéaire ou présente une ramification en position 3 ou plus éloignée par rapport à l'atome fonctionnel d'oxygène, R⁷ représente un groupe alkyle linéaire ou ramifié, comportant de 2 à 12 atomes de carbone, et l'indice b est un nombre entier valant de 1 à 4,
- et le coefficient a prend une valeur de 0 à 2, l'indice n prend une valeur quelconque de 0 à 1, et le donneur est une diamine ou une triamine, étant entendu que a est de préférence un nombre valant entre 0,5 et 1,5 et que n est de préférence le nombre 0 ou 1.

2. Réactifs d'échange conformes à la revendication 1, **caractérisés en ce que** a est un nombre valant entre 0,5 et 1,5 et que n est le nombre 0 ou 1.

3. Réactifs d'échange conformes à la revendication 1 ou 2, **caractérisés en ce qu'**ils se présentent en solution, à une concentration, rapportée au magnésium, d'au moins 0,5 mol/kg, dans un solvant ou un mélange de solvants contenant un hydrocarbure, laquelle solution contient au maximum 1 % en poids d'un solvant de type éther.

4. Réactifs d'échange conformes à l'une des revendications 1 à 3, **caractérisés en ce que** les hydrocarbures sont choisis dans l'ensemble constitué par les aromatiques et les aliphatiques.

5. Procédé de préparation de réactifs d'échange solubles dans des hydrocarbures, conformes à l'une des revendications 1 à 4, **caractérisé en ce que** l'on fait réagir un composé dialcoxy-magnésium R²O-Mg-OR³ avec (n+1) équivalents d'un composé alkyl-lithium R¹Li, dans un solvant ou un mélange de solvants contenant un hydrocarbure, étant entendu que
- R¹ représente une entité choisie dans l'ensemble constitué par les groupes isopropyle (i-Pr), n-butyle (n-Bu), sec-butyle (s-Bu), tert-butyle (t-Bu) et n-hexyle (n-Hex),
- n prend de préférence la valeur 1 ou 0,
- et OR² et OR³ peuvent chacun représenter, indépendamment l'un de l'autre,
e) un groupe alcoxy tertiaire,
f) un groupe alcoxy secondaire,
g) un groupe alcoxy primaire de formule OCH₂CHR⁴R⁵, comportant de 4 à 12 atomes de carbone, lequel groupe alcoxy présente une ramification en position 2 par rapport à l'atome fonctionnel d'oxygène, et dans laquelle formule R⁴ et R⁵ représentent, indépendamment l'un de l'autre, des groupes alkyle comportant de 1 à 8 atomes de carbone,
h) ou un groupe alcoxy portant un autre groupe fonctionnel alcoxy, de formule générale O(CHR⁶)_{b}OR⁷ dans laquelle R⁶ représente un atome d'hydrogène ou un groupe alkyle comportant de 1 à 6 atomes de carbone, qui est linéaire ou présente une ramification en position 3 ou plus éloignée par rapport à l'atome fonctionnel d'oxygène, R⁷ représente un groupe alkyle linéaire ou ramifié, comportant de 2 à 12 atomes de carbone, et l'indice b est un nombre entier valant de 1 à 4.

6. Procédé de préparation de réactifs d'échange solubles dans des hydrocarbures, conformes à l'une des revendications 1 à 4, **caractérisé en ce que** l'on fait réagir un composé dialkyl-magnésium R¹-Mg-R⁹, pour n = 1, avec un équivalent d'un alcool R³OH et avec un équivalent d'un composé alcoxy-lithium R²OLi, ou bien, pour n = 0, avec au total deux équivalents de composés alcoxy-lithium R²OLi et/ou R³OLi, dans un solvant ou un mélange de solvants contenant un hydrocarbure, étant entendu que
- R¹ représente une entité choisie dans l'ensemble constitué par les groupes isopropyle (i-Pr), n-butyle (n-Bu), sec-butyle (s-Bu), tert-butyle (t-Bu) et n-hexyle (n-Hex),
- OR² et OR³ peuvent chacun représenter, indépendamment l'un de l'autre,
a) un groupe alcoxy tertiaire,
b) un groupe alcoxy secondaire,
c) un groupe alcoxy primaire de formule OCH₂CHR⁴R⁵, comportant de 4 à 12 atomes de carbone, lequel groupe alcoxy présente une ramification en position 2 par rapport à l'atome fonctionnel d'oxygène, et dans laquelle formule R⁴ et R⁵ représentent, indépendamment l'un de l'autre, des groupes alkyle comportant de 1 à 8 atomes de carbone,
d) ou un groupe alcoxy portant un autre groupe fonctionnel alcoxy, de formule générale O(CHR⁶)_{b}OR⁷ dans laquelle R⁶ représente un atome d'hydrogène ou un groupe alkyle comportant de 1 à 6 atomes de carbone, qui est linéaire ou présente une ramification en position 3 ou plus éloignée par rapport à l'atome fonctionnel d'oxygène, R⁷ représente un groupe alkyle linéaire ou ramifié, comportant de 2 à 12 atomes de carbone, et l'indice b est un nombre entier valant de 1 à 4,
- et R⁹ représente un groupe alkyle quelconque comportant de 1 à 8 atomes de carbone, ce groupe R⁹ étant identique au groupe R¹ ou différent de celui-ci.

7. Procédé de préparation de réactifs d'échange solubles dans des hydrocarbures, de formule générale R¹MgOR^{3·}LiOR^{2·}a(donneur), conformes à l'une des revendications 1 à 4, **caractérisé en ce que** l'on fait réagir un composé dialkyl-magnésium R¹-Mg-R⁹ avec un équivalent d'un composé dialcoxy-magnésium R⁵O-Mg-OR³, dans un solvant ou un mélange de solvants contenant un hydrocarbure, pour former un mélange réactionnel, et l'on ajoute à ce mélange réactionnel de 0,5 à 1,5 équivalent d'un composé alcoxy-lithium R²OLi, étant entendu que
- R¹ représente une entité choisie dans l'ensemble constitué par les groupes isopropyle (i-Pr), n-butyle (n-Bu), sec-butyle (s-Bu), tert-butyle (t-Bu) et n-hexyle (n-Hex),
- OR² et OR³ peuvent chacun représenter, indépendamment l'un de l'autre,
a) un groupe alcoxy tertiaire,
b) un groupe alcoxy secondaire,
c) un groupe alcoxy primaire de formule OCH₂CHR⁴R⁵, comportant de 4 à 12 atomes de carbone, lequel groupe alcoxy présente une ramification en position 2 par rapport à l'atome fonctionnel d'oxygène, et dans laquelle formule R⁴ et R⁵ représentent, indépendamment l'un de l'autre, des groupes alkyle comportant de 1 à 8 atomes de carbone,
d) ou un groupe alcoxy portant un autre groupe fonctionnel alcoxy, de formule générale O(CHR⁶)_{b}OR⁷ dans laquelle R⁶ représente un atome d'hydrogène ou un groupe alkyle comportant de 1 à 6 atomes de carbone, qui est linéaire ou présente une ramification en position 3 ou plus éloignée par rapport à l'atome fonctionnel d'oxygène, R⁷ représente un groupe alkyle linéaire ou ramifié, comportant de 2 à 12 atomes de carbone, et l'indice b est un nombre entier valant de 1 à 4.

8. Procédé conforme à l'une des revendications 5 à 7, **caractérisé en ce que** l'on ajoute au mélange réactionnel issu de la synthèse a équivalent d'un donneur, par rapport au magnésium, étant entendu que a est de préférence un nombre valant de 0,5 à 1,5.

9. Procédé conforme à l'une des revendications 5 à 8, **caractérisé en ce que** l'on utilise uniquement des solvants à base d'hydrocarbures.

10. Utilisation des réactifs d'échange halogène ou thiolate contre magnésium, solubles dans des hydrocarbures, de formule générale R¹MgR¹₁₋ₙ(OR³)ₙ·LiOR²(1-n)LiOR³·a(donneur), conformes à l'une des revendications 1 à 4 ou obtenus selon l'une des revendications 5 à 9, pour des réactions d'échange avec des composés aromatiques ou hétéroaromatiques halogénés ou porteurs de groupes fonctionnels thiolate, de formules générales Hal-Ar, Hal-HetAr, R⁹S-Ar ou R⁹S-HetAr dans lesquelles R⁹ représente un groupe alkyle ou aryle, et de formation de produits intermédiaires de métallation de formules ArMg-OR^{3·}LiOR^{2·}a(donneur), HetArMg-OR^{3·}LiOR^{2·}a(donneur), Ar₂Mg^{·}LiOR^{2·}LiOR^{3·}a(donneur) et HetAr₂Mg^{·}LiOR^{2·}LiOR^{3·}a(donneur), pour des réactions de couplage CC ou CN ou des réactions d'addition, par conversion à l'aide d'électrophiles.

11. Utilisation des réactifs d'échange halogène ou thiolate contre magnésium, solubles dans des hydrocarbures, de formule générale R¹MgR¹₁₋ₙ(OR³)ₙ^{·}LiOR^{2·}(1-n)LiOR^{3·}a(donneur), conforme à la revendication 10, pour des réactions d'échange avec des composés aromatiques ou hétéroaromatiques halogénés, de formules générales Hal-Ar ou Hal-HetAr, ou avec des composés aromatiques ou hétéroaromatiques porteurs de groupes fonctionnels thiolate, de formules générales R⁸S-Ar ou R⁸S-HetAr, dans le cas où ces composés aromatiques ou hétéroaromatiques, halogénés ou porteurs de groupes fonctionnels thiolate, portent un ou plusieurs groupe(s) fonctionnel(s) supplémentaire(s), choisi(s) dans l'ensemble formé par les suivants : F, Cl, Br, CN, CO₂R, OR, OH, NR₂, NHR, NH₂, PR₂, P(O)R₂, CONR₂, CONHR, SR, SH, CF₃, NO₂.

12. Utilisation des réactifs d'échange halogène ou thiolate contre magnésium, solubles dans des hydrocarbures, de formule générale R¹MgR¹₁₋ₙ(OR³)ₙ^{·}LiOR^{2·}(1-n)LiOR^{3·}a(donneur), conforme à la revendication 11, pour des réactions d'échange avec des composés aromatiques riches en électrons.

13. Utilisation des réactifs d'échange halogène ou thiolate contre magnésium, solubles dans des hydrocarbures, de formule générale R¹MgR¹₁₋ₙ(OR³)ₙ^{·}LiOR^{2·}(1-n)LiOR^{3·}a(donneur), conforme à la revendication 11, pour des réactions d'échange avec des composés hétéroaromatiques riches en électrons, choisis dans l'ensemble formé par les pyrroles, furanes, thiophènes, oxazoles, isoxazoles, thiazoles, isothiazoles, imidazoles, benzimidazoles, triazoles, indazoles et indoles.

14. Utilisation des réactifs d'échange halogène ou thiolate contre magnésium, solubles dans des hydrocarbures, de formule générale R¹MgR¹₁₋ₙ(OR³)ₙ^{·}LiOR^{2·}(1-n)LiOR^{3·}a(donneur), conforme à la revendication 12, pour des réactions d'échange avec des composés aromatiques ou hétéroaromatiques halogénés, de formules générales Hal-Ar ou Hal-HetAr, dans le cas où ces composés aromatiques ou hétéroaromatiques halogénés sont choisis dans l'ensemble formé par les suivants : bromobenzène, bromotoluènes, bromanisoles, bromo-N,N-diméthyl-anilines, 1-bromo-3,5-diméthoxy-benzène, bromonaphtalènes, bromophénanthrènes, bromothiophènes, bromopyridines, bromobenzothiophènes, bromobenzofuranes, 1,2-dibromo-cyclopent-1-ène, 2-(méthyl-thio)-pipéridine-1-carboxylate de tertiobutyle, 2-(phénylthio)-pipéridine-1-carboxylate de tertiobutyle, 4-méthyl-2-(phénylthio)-pipéridine-1-carboxylate de tertiobutyle, 2-[(4-méthoxy-phényl)-thio]-pipéridine-1-carboxylate de tertiobutyle, 2-[(4-fluoro-phényl)-thio]-pipéridine-1-carboxylate de tertiobutyle, 2-(phényl-thio)-pyrrolidine-1-carboxylate de tertiobutyle, et 2-(phényl-thio)-pyridine.
